(19) **Europäisches Patentamt / European Patent Office / Office européen des brevets**

(11) **EP 4 278 016 B1**

(12) **EUROPEAN PATENT SPECIFICATION**

(45) Date of publication and mention
of the grant of the patent:
**20.05.2026   Bulletin 2026/21**

(21) Application number: **22701211.9**

(22) Date of filing: **17.01.2022**

(51) International Patent Classification (IPC):
***C12Q 1/6886*** (2018.01)

(52) Cooperative Patent Classification (CPC):
**C12Q 1/6886;** C12Q 2600/112; C12Q 2600/118;
C12Q 2600/158

(86) International application number:
**PCT/EP2022/050915**

(87) International publication number:
**WO 2022/152911 (21.07.2022 Gazette 2022/29)**

(54) **METHOD FOR THE PREDICTION, DETECTION, STRATIFICATION, MONITORING, AND FOLLOW-UP OF BREAST CANCER AT ANY STAGE OF THE DISEASE**

VERFAHREN ZUR VORHERSAGE, ERKENNUNG, STRATIFIZIERUNG, ÜBERWACHUNG UND NACHSORGE VON BRUSTKREBS IN JEDEM STADIUM DER KRANKHEIT

PROCÉDÉ PERMETTANT DE PRÉVOIR, DÉTECTER, STRATIFIER, SURVEILLER ET SUIVRE LES CANCERS DU SEIN À TOUT STADE DE LA MALADIE

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: **15.01.2021   EP 21151781**

(43) Date of publication of application:
**22.11.2023   Bulletin 2023/47**

(73) Proprietor: **Université de Fribourg**
**1700 Fribourg (CH)**

(72) Inventors:
• **RUEGG, Curzio**
**1630 Bulle (CH)**
• **CATTIN, Sarah**
**1636 Broc (CH)**

(74) Representative: **Jelsch, Emmanuel Edwin**
**IP2Go-Pro**
**Rue de Lausanne 64**
**1020 Renens (VD) (CH)**

(56) References cited:
EP-A1- 2 836 838      WO-A1-2010/063454
WO-A1-2017/011559

• CATTIN SARAH ET AL: "Circulating immune cell populations related to primary breast cancer, surgical removal, and radiotherapy revealed by flow cytometry analysis", BREAST CANCER RESEARCH : BCR, 5 June 2021 (2021-06-05), London, pages 1 - 13, XP055826756, [retrieved on 20210722], DOI: 10.1186/s13058-021-01441-8
• ANONYMOUS: "Affymetrix GeneChip Human Genome U133 Array Set HGU133A", NCBI, GEO, PLATFORM  GPL96, 11 March 2002 (2002-03-11), pages 1 - 511, XP055546924, Retrieved from the Internet <URL:https://www.ncbi.nlm.nih.gov/geo/query/acc.cgi?acc=GPL96> [retrieved on 20190124]
• PENG YUN ET AL: "miR-301b and NR3C2 co-regulate cells malignant properties and have the potential to be independent prognostic factors in breast cancer", JOURNAL OF BIOCHEMICAL AND MOLECULAR TOXICOLOGY, vol. 35, no. 2, 15 October 2020 (2020-10-15), US, XP55913503, ISSN: 1095-6670, Retrieved from the Internet <URL:https://onlinelibrary.wiley.com/doi/full-xml/10.1002/jbt.22650> DOI: 10.1002/jbt.22650

**(Cont. next page)**

- **SONG GUO-DONG ET AL: "SOX4 overexpression is a novel biomarker of malignant status and poor prognosis in breast cancer patients", TUMOR BIOLOGY, KARGER, BASEL, CH, vol. 36, no. 6, 16 January 2015 (2015-01-16), pages 4167 - 4173, XP035527437, ISSN: 1010-4283, [retrieved on 20150116], DOI: 10.1007/S13277-015-3051-9**
- **PENG YUN ET AL: "miR-301b and NR3C2 co-regulate cells malignant properties and have the potential to be independent prognostic factors in breast cancer", JOURNAL OF BIOCHEMICAL AND MOLECULAR TOXICOLOGY, vol. 35, no. 2, 15 October 2020 (2020-10-15), US, XP055913503, ISSN: 1095-6670, Retrieved from the Internet <URL:https://onlinelibrary.wiley.com/doi/full-xml/10.1002/jbt.22650> DOI: 10.1002/jbt.22650**
- **NAGARAJAN DIVYA ET AL: "Immune Landscape of Breast Cancers", BIOMEDICINES, vol. 6, no. 1, 11 February 2018 (2018-02-11), pages 20, XP055826768, DOI: 10.3390/biomedicines6010020**
- **GIRIECA LORUSSO ET AL: "The tumor microenvironment and its contribution to tumor evolution toward metastasis", HISTOCHEMISTRY AND CELL BIOLOGY, SPRINGER, BERLIN, DE, vol. 130, no. 6, 6 November 2008 (2008-11-06), pages 1091 - 1103, XP019657787, ISSN: 1432-119X, DOI: 10.1007/S00418-008-0530-8**
- **B. RUFFELL ET AL: "Leukocyte composition of human breast cancer", PROCEEDINGS OF THE NATIONAL ACADEMY OF SCIENCES, vol. 109, no. 8, 8 August 2011 (2011-08-08), pages 2796 - 2801, XP055132332, ISSN: 0027-8424, DOI: 10.1073/pnas.1104303108**

Remarks:
    The file contains technical information submitted after the application was filed and not included in this specification

**Description**

**FIELD OF THE INVENTION**

The invention relates to a method to detect, predict, stratify, monitor and follow-up breast cancers in a female subject. Also disclosed is a breast cancer detection kit for use in the detection, stratification, monitoring and follow-up of breast cancer in a female subject.

**BACKGROUND OF THE INVENTION**

Currently, the state of the art in the early detection of breast cancer is based on imaging methods, in particular mammography. No alternative of validated biochemical or molecular tests exists today. For formal diagnosis an invasive biopsy is required.

Histological, genetic and transcriptomic analyses are performed on tumor material obtained at surgery to determine tumor subtypes and stratify patients for optimal therapy. For the follow-up, patients are monitored non-specifically by clinical, hematological, biochemical and molecular tests, mostly to detect unwanted effects of chemotherapy. Relapses are largely detected indirectly, upon clinical symptoms or non-specific laboratory results.

**Breast cancer** is a well-know and well-described pathology affecting approximately 12% of women over an 80-year lifespan. In total, roughly 2 million new cases per year are diagnosed world-wide (nearly 500'000 in Europe and 6'000 in Switzerland). Breast cancer prognosis and therapy are largely determined by the biological and molecular characteristics of the tumor and the stage at diagnosis. Three main clinically relevant subtypes of breast cancer have been defined: Estrogen receptor positive ($ER^+$), often also progesterone receptor positive ($PR^+$), Human Epidermal Growth Factor Receptor 2 amplified ($HER2^+$) and triple negative breast cancer (TNBC; i.e. $ER^-$, $PR^-$ and $HER2^-$ breast cancers). Molecular subtypes (e.g. Luminal A, Luminal B, $HER2^+$, basal-like) that overlap largely, but not fully with the clinical subtypes, have been defined based on gene expression profiling [1]. Adjuvant treatments, in combination with mastectomy or breast-saving surgery, have improved survival by about 30%, in particular with the introduction or anti-estrogen (e.g. tamoxifen) and anti-HER2 (e.g. Herceptin) based-treatments, for $ER^+$ and $HER2^+$ cancers, respectively. For TNBC there are still no specific therapies due to lack of defined targets, and radio- and chemotherapies are routinely used instead. Further improvements of adjuvant treatments are becoming rare and are mostly dependent on the identification of patients responding to available treatments [2]. The development of therapy-resistant metastases in vital organs, in particular liver, lung, bones and brain leading to organ disruption and failure, is the ultimate cause of death in relapsing patients. As curative therapies are largely lacking once metastases have formed, a significant improvement in the survival of breast cancer patients can only be achieved by developing treatments controlling metastatic disease or improving detection at a preinvasive state.

**Breast cancer gene expression signatures:** Gene expression profiling from tumor biopsies classified breast cancer into different molecular subtypes with distinct features and clinical outcomes: luminal A, luminal B, HER2-enriched, basal-like, claudin-low and normal-like subtypes [1]. This molecular classification contributed to refine the diagnosis and selection of the most appropriate therapy. More recently, gene expression signatures (commercialized as Mammaprint and Oncotype DX) have been used, in conjunction with clinical-pathological parameters, to predict response to chemotherapy with the intent to avoid overtreatment of patients with good prognosis [3]. These signatures, however cannot be applied for early detection or follow up of patients as they are based on the analysis of tumor tissue.

**Immune-inflammatory response in breast cancer:** Inflammatory cells and the innate immune system play an important role in controlling tumor progression and metastasis mainly through the recruitment of $CD11b^+$ cells in the tumor and (pre-) metastatic microenvironments, including in breast cancer [4]. $CD11b^+$ monocytes are attracted to tumor sites by inflammatory cytokines, chemokines and angiogenic factors and polarize toward an alternative activation state, referred to as M2, in contrast to the classical M1 activation state. M1 macrophages possess cytotoxic and tumor-suppressive activities, while M2 macrophages have tumor-promoting and immunosuppressive activities. Diverse tumor-promoting $CD11b^+$ cell types have been reported, including: Vascular Endothelial Growth Factor receptor $1^+$ ($VEGFR-1^+$) $CD11b^+$, $Gr1^+CD11b^+$ myeloid-derived suppressor cells (MDSC), Tie-2-expressing $CD11b^+$ monocytes (TEM) and $cKit^+CD11b^+$ cells. Tumor infiltrating lymphocytes (TILs) play an increasingly recognized role in controlling progression and clinical outcome in breast cancer. TNBC presents the richest presence of Tumor Infiltrating Lymphocytes (TILs), $CD8^+$ T-cell infiltrates and tertiary lymphoid structures. In TNBC, increased number of TIL is associated with improved pathological complete response following neoadjuvant therapy [5]. Elevated TILs levels correlate with decreased rates of distant recurrences and improved survival. Enumeration of TILs in breast cancer (as part of an Immunoscore evaluation) has been recommended as an immunological biomarker with prognostic and potentially predictive values, especially in TNBC and HER2-amplified breast cancers [5].

**Breast cancer detection and monitoring:** The current screening test for breast cancer is mammography, which is usually made once every 2 years stating at the age of 50. The main limitation of mammography is that it is based on morphology, hence on the detection of a sizable tumor. Additional imaging-based techniques (i.e. computer tomography (CT), Magnetic Resonance Imaging (MRI), Sonography) may be used in complement to mammography. A biopsy is then necessary for formal diagnosis. In some countries the necessary infrastructure is limited, so that this screening test is seldomly applied, rendering the early detection difficult. Mammography screening, however, has important limitations the main being that it is based on morphology, hence on the detection of a sizable tumor. Mammography particularly detects ductal carcinoma *in situ* (DCIS) or lobular carcinoma *in situ* (LCIS), non-invasive cancers, leading to over-treatments: for 1000 women screened over 20 years only 5 deaths are prevented while 17 patients are overtreated [6]. Because of overtreatment the benefit of mammography is currently under scrutiny. Mammography also often misses interval cancers, which are rapidly growing and invasive.

Alternative, methods not based on morphology, are under evaluation, including the dection of circulating tumor cells (CTC) or genetic material such as circulating turmoral DNA (ctDNA), micro RNA (miRNA), other RNAs in the systemic circulation ("liquid biopsy"). These approaches require the presence of a significant tumor mass releasing matierial into the systemic circulation at sufficient amounts allowing detection with current techniques. ctDNA-based approaches are based the detection of cancer-associated mutations, copy number variations or epigenetic modifications (e.g. methylation) specifc for a given cancer, thereby limiting sensitivity and possibly specificity for early detection purposes [7]. The interest in CTC to develop clinical tests has been hampered due to technical difficulties in their detection (very rare events; limited cell surface markers; multistep technologies) and their rather late presence during disease progression. None of these approaches have reached clinical routine yet.

Basically, all the current blood-based tests are based on markers generated by the tumor itself, and thus dependent on an already developed and vascularized tumors. Thus, the detection of microscopic primary tumor or metastatic lesions remains a challenge and an unmet need in oncology.

It is well-known that early cancer detection is associated with increased chances of cure, while the later the cancer is detected, the lower the probability of cure and survival. Waiting for the tumor to be of significant size renders the treatment more difficult and reduces the probability of full cure.

The same is true for metastatic cancer appearing after an initial treatment. The earlier the metastasis is detected, the more likely a therapy will be successful in controlling further tumor evolution. Novel, effective therapies for metastatic breast cancer are being tested and introduced in clinical routine.

After initial treatment (i.e. surgery, adjuvant therapy), disease follow-up is currently largely based on clinical examination and general laboratory analyses performed at regular intervals. Imaging-based detection of metastases is complicated due to the fact that cancer cells can disseminate to all major organs of the body, and metastases may appear in multiple organs, rendering a reliable imaging-based early detection of relapses virtually impossible. There are no validated, clinically established targeted tests, biomarkers or procedures to specifically assess whether the disease is cured, dormant or progressive. CTC, ctDNA, miRNA and other circulating tumor-derived moieties have been intensively investigated as candidate monitoring tools but so far, no routine test based upon has been validated [8].

WO2014068124 A1 (NOVIGENIX SA) discloses methods for the detection of predetermined biomarkers for early diagnosis and management of colorectal tumors, wherein the biomarkers are selected from IL1B, PTGS2, S100A8, LTF, CXCL10, CACNB4, MMP9, CXCL11, EGRI, JUN, TNFSF13B, GATA2, MMPII, NMEI, PTGES, CCRI, CXCR3, FXYD5, IL8, ITGA2, ITGBS, MAPK6, RHOC, BCL3, CD63, CESI, MAP2K3, MSLI, and PPARG.

Nagarajan Divya et al: "Immune Landscape of Breast Cancers", Biomedicines, vol. 6, no. 1, 11 February 2018 (2018-02-11), page 20, XP055826768, DOI: 10.3390/biomedicines6010020, disclose that breast cancer is a very heterogeneous disease, both at molecular and histological levels. Five intrinsic subtypes were initially identified-Luminal-A, Luminal-B, HER2[+], Triple negative/basal like (TNBC) and normal like-subsequently expanded to seven (Basal-like-1 and 2, mesenchymal, mesenchymal stem-like, luminal androgen receptor, immuno-modulatory and unstable). Although genetic and epigenetic changes are key pathogenic events, the immune system plays a substantial role in promoting progression and metastasis. This review discusses the extent to which immune cells can be detected within the tumor microenvironment, as well as their prognostic role and relationship with the microbiome, with an emphasis on TNBC.

Girieca Lorusso et al: "The tumor microenvironment and its contribution to tumor evolution toward metastasis", histochemistry and cell biology, Springer, Berlin, De, vol. 130, no. 6, 6 November 2008 (2008-11-06), pages 1091-1103, XP019657787, ISSN: 1432-119X, DOI: 10.1007/S00418-008-0530-8 disclose that cancer cells acquire cell-autonomous capacities to undergo limitless proliferation and survival through the activation of oncogenes and inactivation of tumor suppressor genes. Nevertheless, the formation of a clinically relevant tumor requires support from the surrounding normal stroma, also referred to as the tumor microenvironment. Carcinoma-associated fibroblasts, leukocytes, bone marrow-derived cells, blood and lymphatic vascular endothelial cells present within the tumor microenvironment contribute to tumor progression. Recent evidence indicates that the microenvironment provides essential cues to the maintenance of cancer stem cells/cancer initiating cells and to promote the seeding of cancer cells at metastatic sites. Furthermore, inflammatory cells and immunomodulatory mediators present in the tumor microenvironment polarize host immune response toward specific phenotypes impacting tumor progression. A growing number of studies demonstrate a positive correlation between angiogenesis, carcinoma-associated fibroblasts, and inflammatory infiltrating cells and poor outcome, thereby emphasizing the clinical relevance of the tumor microenvironment to aggressive tumor progression. Thus, the dynamic and reciprocal interactions between tumor cells and cells of the tumor microenvironment orchestrate events critical to tumor evolution toward metastasis, and many cellular and molecular elements of the microenvironment are emerging as attractive targets for therapeutic strategies.

In B. Ruffell et al: "Leukocyte composition of human breast cancer", Proceedings of the National Academy of Sciences, vol. 109, no. 8, 8 August 2011 (2011-08-08), pages 2796-2801, XP055132332, ISSN: 0027-8424, DOI: 10.1073/pnas.1104303108 it is disclosed that retrospective clinical studies have used immune-based biomarkers, alone or in combination, to predict survival outcomes for women with breast cancer (BC); however, the limitations inherent to immunohistochemical analyses prevent comprehensive descriptions of leukocytic infiltrates, as well as evaluation of the functional state of leukocytes in BC stroma. To more fully evaluate this complexity, and to gain insight into immune responses after chemotherapy (CTX), authors prospectively evaluated tumor and nonadjacent normal breast tissue from women with BC, who either had or had not received neoadjuvant CTX before surgery. Tissues were evaluated by polychromatic flow cytometry in combination with confocal immunofluorescence and immunohistochemical analysis of tissue sections. These studies revealed that activated T lymphocytes predominate in tumor tissue, whereas myeloid lineage cells are more prominent in "normal" breast tissue. Notably, residual tumors from an unselected group of BC patients treated with neoadjuvant CTX contained increased percentages of infiltrating myeloid cells, accompanied by an increased CD8/CD4 T-cell ratio and higher numbers of granzyme B-expressing cells, compared with tumors removed from patients treated primarily by surgery alone. These data provide an initial evaluation of differences in the immune microenvironment of BC compared with nonadjacent normal tissue and reveal the degree to which CTX may alter the complexity and presence of selective subsets of immune cells in tumors previously treated in the neoadjuvant setting.

WO 2017/011559 A1 (MEDIMMUNE LLC [US]) 19 January 2017 (2017-01-19) provides compositions and methods that are useful in the treatment of cancer. More specifically, the methods and compositions may be used to detect, quantify, inhibit, kill, differentiate, or eliminate cancer stem cells (CSCs) and may be used in the treatment of cancers associated with CSCs, and particularly cancers and CSCs that express CCL20 and/or CCR6.

WO 2008/123867 A1 (SOURCE PRECISION MEDICINE INC [US]; WASSMANN KARL [US] ET AL.) 16 October 2008 (2008-10-16) provides a method in various embodiments for determining a profile data set for a subject with breast cancer or conditions related to breast cancer based on a sample from the subject, wherein the sample provides a source of RNAs. The method includes using amplification for measuring the amount of RNA corresponding to at least 1 constituent from Tables 1-5. The profile data set comprises the measure of each constituent, and amplification is performed under measurement conditions that are substantially repeatable.

In 2016 Cattin *et al.;* ref [9] reported that human metastatic BC (mBC) had elevated frequencies of circulating endothelial cells/endothelial progenitors, TIE2$^+$CD11b$^+$ and CD117(KIT)$^+$CD11b$^+$ cells. Cancer patients expressed higher mRNA levels of the M2 polarization markers CD163, Arginase 1 (ARG1) and Interleukin-10 (IL-10) in CD11b$^+$ cells and increased plasma levels of IL-10 and CCL20, two factors produced by alternatively activated (M2) monocytes/macrophages. In contrast, activation markers and cytokines of inflammatory monocytes/macrophages (M2) were low or equally expressed in cancer patients compared to healthy donors. Transcriptome of CD11b$^+$ myeloid cells derived from four mBC patients and four healthy donors (HD) revealed that circulating CD11b$^+$ cells in mBC patients have a distinctive gene expression profile compared to HD. The authors identified 271 genes significantly differentially expressed between mBC patients and HD. Interestingly, mBC patients expressed genes of M2 monocytes' activation state (i.e. IL-10 and CCL20). Taken together, these data demonstrated that circulating CD11b$^+$cells in mBC patients have a different phenotypical (cell surface) and transcriptional profiles compared to CD11b$^+$ cells in healthy individuals.

SONG GUO-DONG ET AL: "SOX4 overexpression is a novel biomarker of malignant status and poor prognosis in breast cancer patients", TUMOR BIOLOGY, KARGER, BASEL, CH, vol. 36, no. 6, 16 January 2015 (2015-01-16), pages 4167-4173, XP035527437, ISSN: 1010-4283, DOI: 10.1007/S13277-015-3051-9 discloses that sex-determining region Y-related high mobility group box 4 (SOX4) has been proven to serve as a critical role in cancer development and progression. The purpose of this study was to measure the expression of SOX4 in breast cancer patients and to explore the clinical significance of SOX4. Using RT-PCR and Western blot, messenger RNA QIRNA) and protein expression of SOX4 were measured in breast cancer tissues and adjacent normal mammary tissues. The relationship of SOX4 expression with clinical characteristics of 148 breast cancer patients was analyzed by immunohistochemistry. In the study, the results indicated that SOX4 mRNA and protein were highly expressed in breast cancer tissues compared with adjacent normal mammary tissues and positively correlated with clinical stage (I-II vs. III-IV; P:0.008), T classification (TI-T2 vs. T3-T4; P:0.013), N classification (N0-N1 vs. N2-N3; P<0.001), M classification (M0 vs. M1; P:0.001), estrogen receptor (negative vs. positive: P:0.029), progesterone receptor (negative vs. positive; P:0.004), and histological grade (G1 vs. G2-G3, P:0.033) in breast cancer patients. Furthermore, they also found that SOX4 protein overexpression was an unfavorable prognostic factor in breast cancer patients (P<0.001), regardless of clinical stage, tumor size, lymph node metastasis, and distant metastasis. Finally, high SOX4 expression was an independent poor prognostic factor for pancreatic patients through multivariate analysis (P:0.033). In conclusion, SOX4 overexpression serves as an unfavorable prognostic biomarker in breast cancer patients.

In summary, the detection of microscopic primary tumor or metastatic lesions remains a challenge and an unmet need in oncology. BRIEF DESCRIPTION OF THE INVENTION The invention mainly uses blood samples to detect biomarkers generated by the immune system when cancer cells are encountered. Surprisingly, using the reaction of the organism toward cancer instead of the signals created by the cancer enables an early and sensitive detection of the cancer. The present invention is partially based upon the discovery that a small panel of biomarkers in the blood is able to specifically identify and distinguish subjects with breast cancer or precancerous lesions from subject without such lesions. Accordingly, the invention provides unique advantages to the female patient associated with early detection of breast cancer or tumor in the patient, including increased life span, decreased morbidity and mortality, decreased exposure to radiation during screening and repeat screenings and a minimally invasive diagnostic model. Importantly, the methods of the invention allow for a patient to avoid invasive procedures, thus increasing patient's compliance. The invention is a combination of the variations of expression levels of genetic (transcriptome) and cell surface biomarkers specific for the immune response elicited by breast cancer at its different stages. In summary, the invention relates to the detection of breast cancer in a female subject, whereby:

(1.) the transcriptomic expression level of a marker panel of 5 genes (SOX4, TNFSF10, CD3G, and NR3C2) and, secondly,

(2.) a panel of cell surface markers, altogether 7 ( CD11b, CD62L, CD86, CD117, CD144, CD177, and CD202b) are measured in their expression level, where subsequently a probability score is calculated based on the measurement of the transcriptomic expression of both said marker panels and where a pre-determined score decides on whether breast cancer can be ruled out or in.

In particular, one of the objects of the present invention is to provide a breast cancer detection method for a female subject, comprising:

(a) measuring in a biologic sample obtained from said female subject the expression level of transcriptomic (mRNA) markers of a first panel comprising the combination of SOX4, TNFSF10, CD3G, and NR3C2, and cell surface biomarkers specific for the immune response elicited by breast cancer at its different stages of a second panel comprising the combination of CD11b, CD62L, CD86, CD117, CD144, CD177, and CD202b;
(b) calculating a probability score based on the measurement of step (a); and
(c) ruling out breast cancer for said female subject if the score in step (b) is lower than a predetermined score; or
(d) ruling in the likelihood of breast cancer for said female subject if the score in step (b) is higher than a pre-determined score.

Another object of the present invention is to provide a breast cancer classifying or stratifying prognostic method for classifying/stratifying whether a female subject is more likely to develop breast cancer comprising:

a) measuring in a biologic sample obtained from said female subject the expression level of transcriptomic (mRNA) markers of a first panel comprising the combination of SOX4, TNFSF10, CD3G, and NR3C2, and cell surface biomarkers specific for the immune response elicited by breast cancer at its different stages of a second panel comprising the combination of CD11b, CD62L, CD86, CD117, CD144, CD177, and CD202b;
(b) comparing the amount measured in step (a) to a reference value; and
(c) classifying the female subject as more likely to have progredient breast cancer when an increase or a decrease in the amount of each transcriptomic (mRNA) markers of the first panel and in the amount of at least one protein biomarker of the second panel relative to a reference value is detected in step (b).

A further object of the invention is to provide a breast cancer detection kit for use in the detection, stratification and/or monitoring of a likelihood of breast cancer in a female subject from a peripheral blood sample, said kit comprising:

- at least one probe for measuring transcriptomic (mRNA) marker of a first panel comprising the combination of SOX4, TNFSF10, CD3G, and NR3C2
- at least one probe and/or specific detection reagent for measuring one or more cell surface biomarker specific for the immune response elicited by breast cancer at its different stages of a second panel comprising the combination of CD11b, CD62L, CD86, CD117, CD144, CD177, and CD202b.

Other objects and advantages of the invention will become apparent to those skilled in the art from a review of the ensuing detailed description, which proceeds with reference to the following illustrative drawings, and the attendant claims.

## BRIEF DESCRIPTION OF THE FIGURES

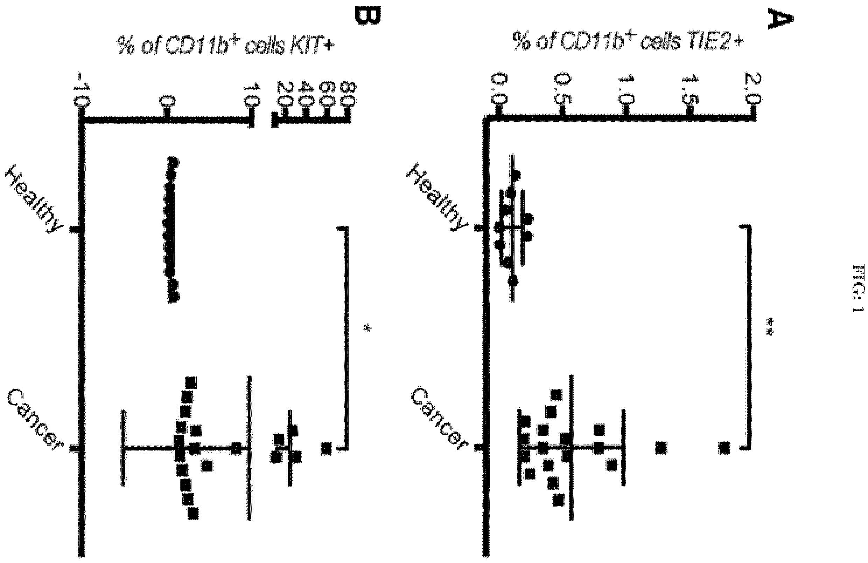

**Figure 1:** shows increased frequency of TIE2[+]CD11b[+] (A) and KIT+CD11b[+] (B) cells in the blood of metastatic breast cancer patients and healthy donors. Cell analysis and quantification was performed by flow cytometry with FlowJo software and results are represented as mean values +/- SD [9].

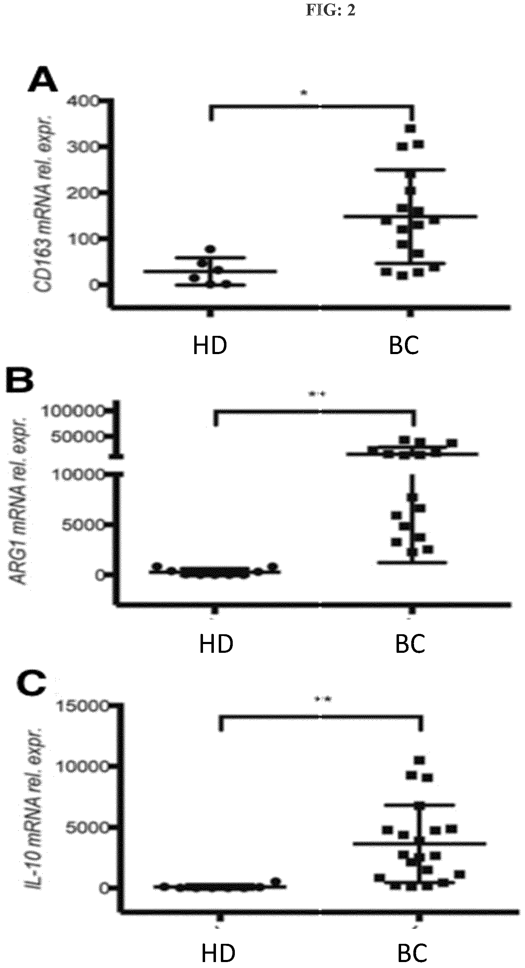

**Figure 2:** demonstrates a M2 activation phenotype of CD11b[+] cells in the blood of metastatic breast cancer patients. (A) Quantification of the mRNA expression levels of M2 markers CD163, (B) ARG1 and (C) IL-10 in CD11b[-] cells derived from the blood of healthy donors compare to cancer patients before therapy start. Analysis was performed by real time qPCR. All data are represented as mean +/- SD [9].

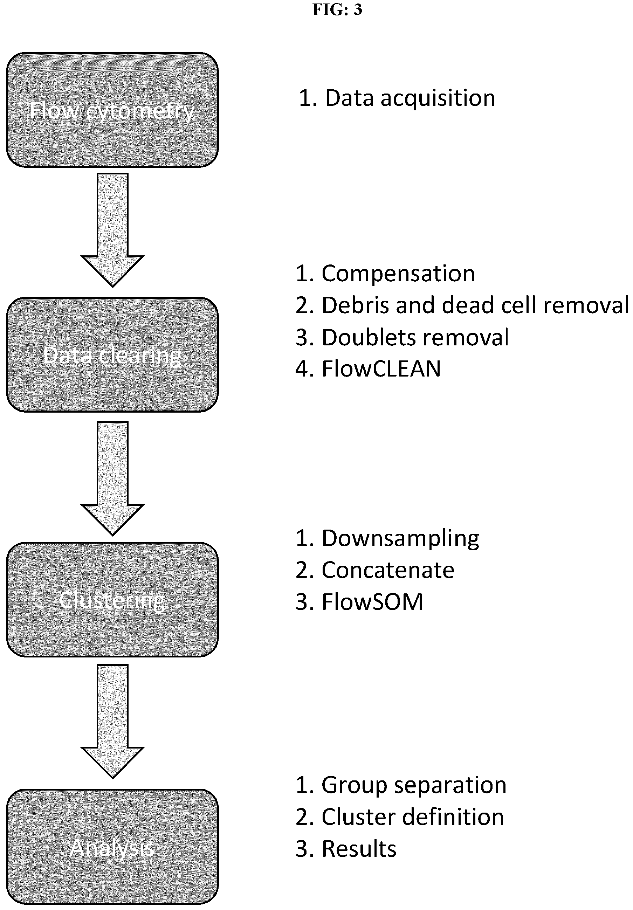

**Figure 3:** shows the workflow for unsupervised analysis of flow cytometry data. The analytical process consists of three different steps (data cleaning; data clustering and data analysis) applied to the flow cytometry raw data for reproducible analysis in time, avoid investigator-associated bias and identify unanticipated cell populations.

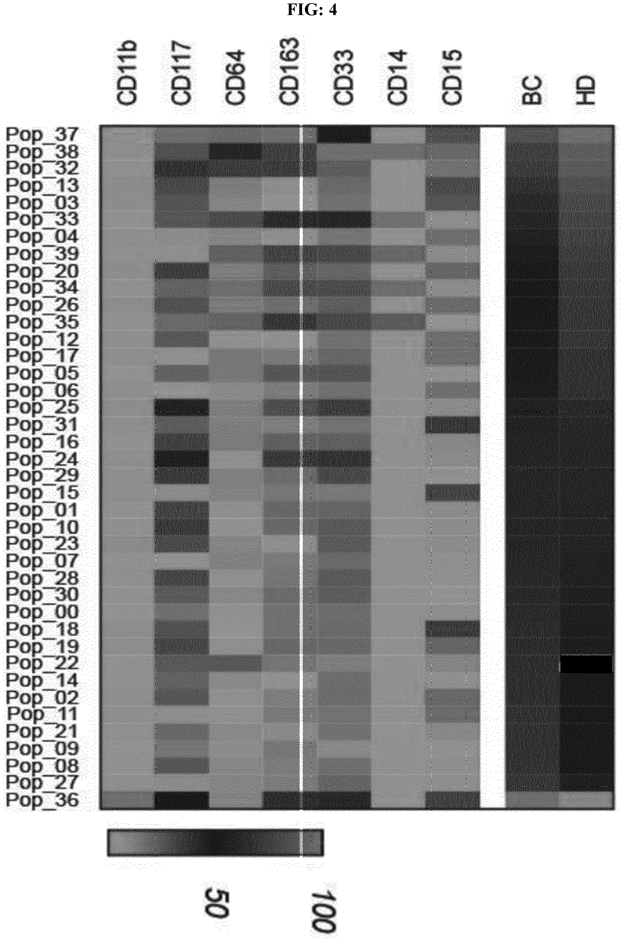

**Figure 4:** illustrates altered frequency of circulating monocytic populations in cancer patients. Heat map of the FlowSOM clustering between breast cancer patients (BC) and healthy donors (HD). Cell analysis and quantification was performed by flow cytometry with FlowJo software and results are represented as mean values +/- SD.

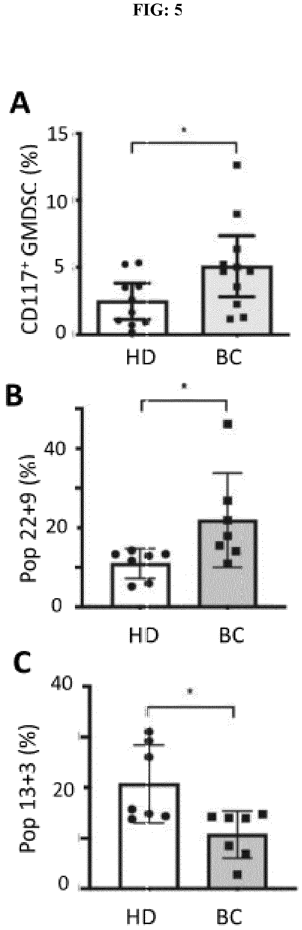

**Figure 5:** illustrates altered frequency of circulating monocytic populations in cancer patients. Frequency of (A) CD117[+] Granulocytic (G)-MDSC population, and the atypical populations (B) 22+9 and (C) 13+3 at the same timing. Cell analysis and quantification was performed by flow cytometry with FlowJo software and results are represented as mean values +/- SD.

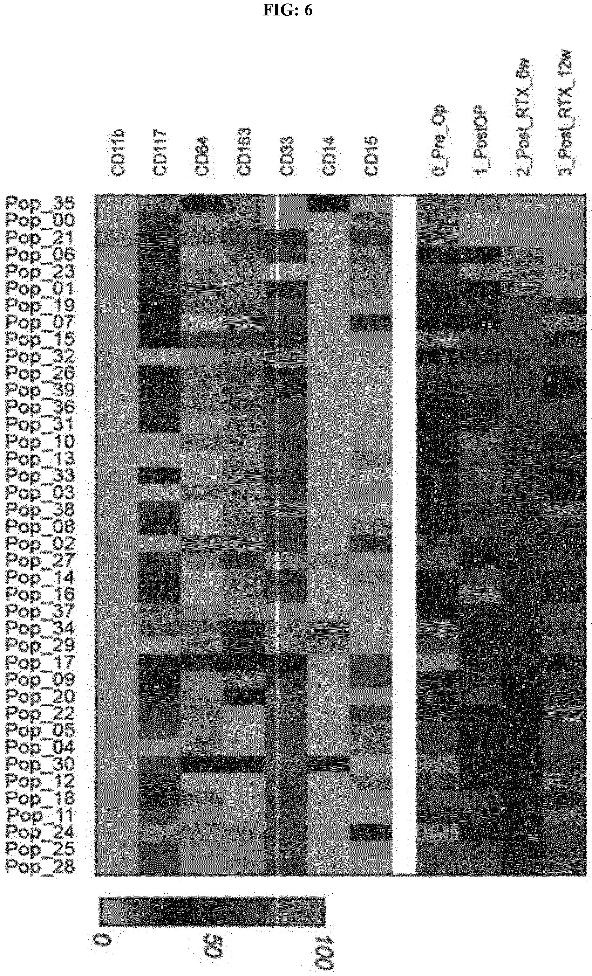

**Figure 6:** shows the heat map of the FlowSOM clustering of breast cancer patients at indicated time-points. Frequency of (B) Monocytic (Mo)-MDSC and (C) G-MDSC cell populations in patients at the indicated time points during treatment. 0_PreOp time- point. Cell analysis and quantification was performed by flow cytometry with FlowJo software and results are represented as mean values +/- SD.

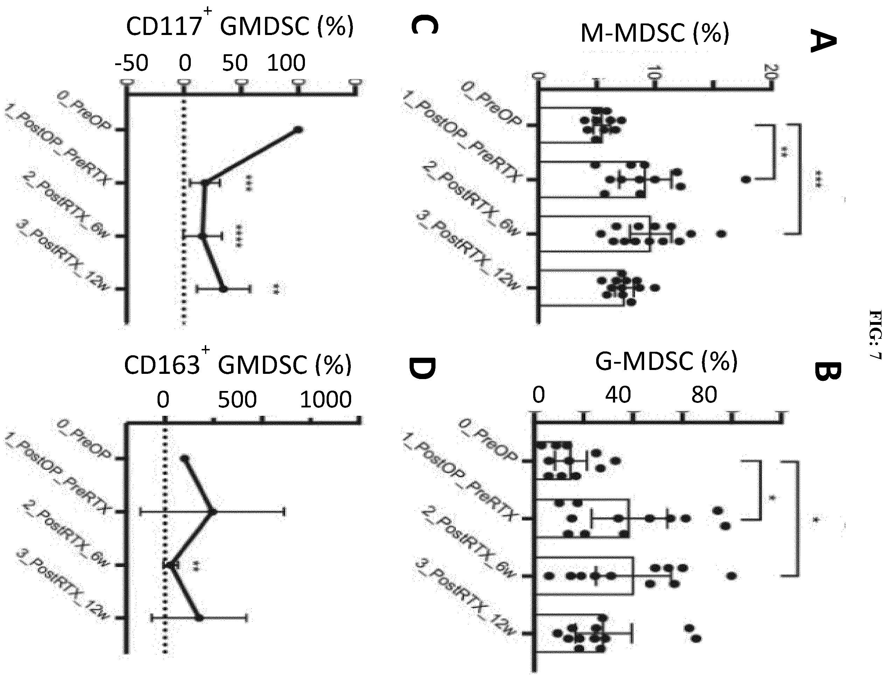

**Figure 7:** shows that tumor removal reduces the frequency of circulating CD117[+] G-MDSC cells. Frequency of (A) Mo-MDSC; (B) G-MDSC; C) CD163[+]; (D) CD117[+] G-MDSCs frequency of (population at indicated time-points relative to frequency at 0_PreOp time- point. Analysis and quantification were performed by flow cytometry with FlowJo software and results are represented as mean values +/- SD.

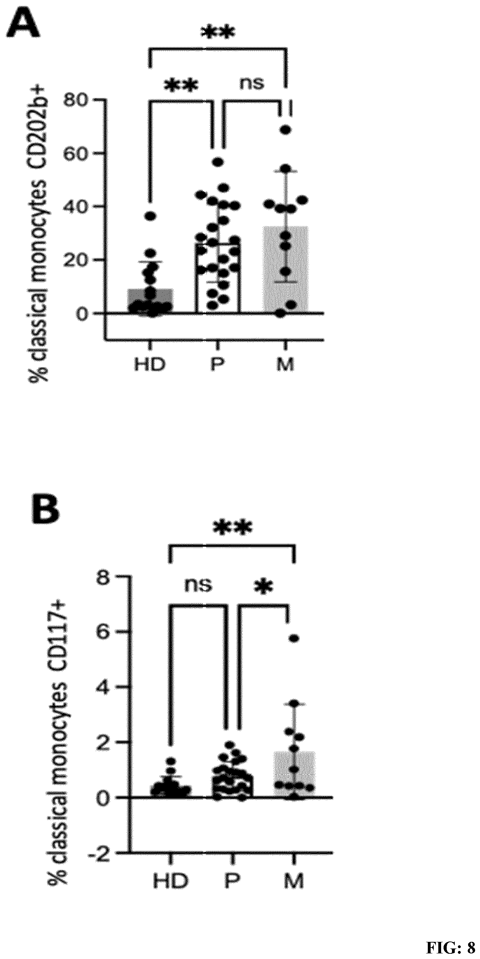

**Figure 8:** shows increased frequency of CD202b[+] (TIE2) CD11b[+] classical monocytes (A), and CD117[+] (C-KIT) CD11b[+] classical monocytes (B) in the blood breast cancer patients with primary BC (P), metastatic BC (M) and healthy donors (HD). Cell analysis and quantification were performed by flow cytometry and FlowJo software. Results are represented as mean values +/- SD (See Example 3).

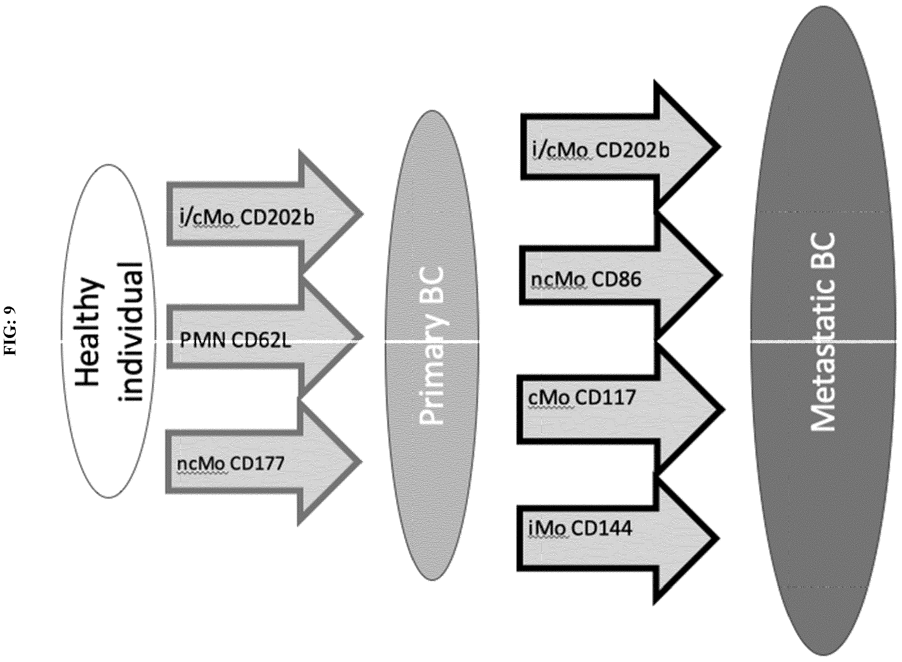

**Figure 9:** Example of a procedure to identify primary BC and metastatic BC. Primary BC patients can be identified through increased levels of CD62L on neutrophil polymorphonuclear (PMN) granulocytes, increased levels of CD202b on intermediate and classical monocytes (icMo) and decreased expression of CD177 on non-classical monocytes (ncMo). Metastatic BC patients can be identified through increased levels of CD202b on intermediate and classical monocytes (icMo), increased levels of CD117 on classical monocytes (cMo), increased levels of CD144 on intermediated monocytes (iMo) and decreased levels of CD86 on non-classical monocytes (ncMo) (See Example 3).

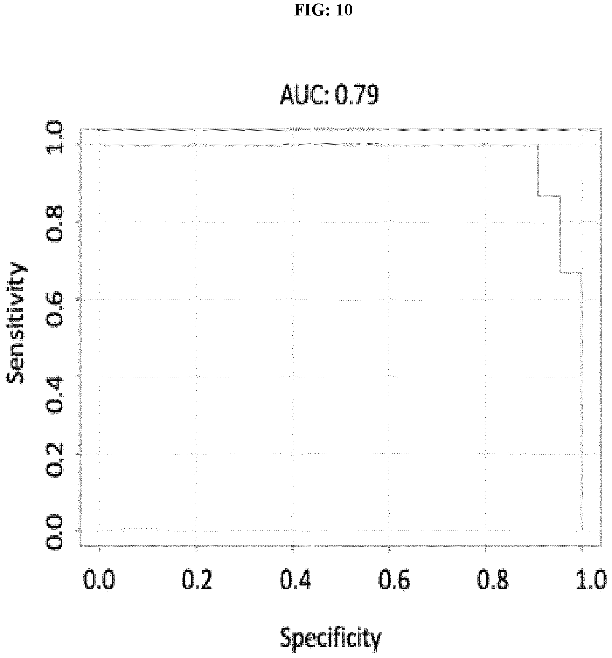

**Figure 10:** Example of ROC curve of the complete logistic model against the real classification values of the patients (healthy donors vs patients with primary breast cancer) representing the specificity (1 - false positive rate (FPR)) against the sensitivity (true positive rate (TPR)) at various thresholds.

## DETAILED DESCRIPTION OF THE INVENTION

The present invention mainly uses blood samples to detect biomarkers generated by the immune system when cancer cells are encountered. One object of the invention relates to a method to detect, predict, stratify, monitor and follow-up breast cancers at any stage of the disease. The invention also discloses a breast cancer detection kit directed to the detection, stratification, monitoring and follow-up of breast cancer in a female subject.

One particular advantage of the present invention is, for example, to identify early stage breast cancer in a female subject by means of expression markers.

Surprisingly, the invention provides for the identification of early stage breast cancer by means of expression profiling:

(1.) the transcriptomic expression level of a marker panel of 5 genes (SOX4, TNFSF10, CD3G, and NR3C2) and, secondly,

(2.) a panel of cell surface markers, altogether 7 ( CD11b, CD62L, CD86, CD117, CD144, CD177, and CD202b) are measured in their expression level, where subsequently a probability score is calculated based on the measurement of the transcriptomic expression of both said marker panels and where a pre-determined score decides on whether breast cancer can be ruled out or in.

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control.

Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components.

As used in the specification and claims, the singular forms "a", "an" and "the" include plural references unless the context clearly dictates otherwise.

"Treating" or "treatment" as used herein with regard to a condition may refer to preventing the condition, slowing the onset or rate of development of the condition, reducing the risk of developing the condition, preventing or delaying the development of symptoms associated with the condition, reducing or ending symptoms associated with the condition, generating a complete or partial regression of the condition, or some combination thereof.

A "biomarker" used herein refers to a molecular indicator of a specific biological property; a biochemical feature or facet that can be used to detect breast cancer. "Biomarker" encompasses, without limitation, proteins, nucleic acids, and metabolites, together with their polymorphisms, mutants , isoform variants, related metabolites, derivatives, precursors including nucleic acids and pro-proteins, cleavage products, protein-ligand complexes, post-translationally modified variants (such as cross-linking or glycosylation), fragments, and degradation products, as well as any multi-unit nucleic acid, protein, and glycoprotein structures comprised of any of the biomarkers as constituent subunits of the fully assembled structure, and other analytes or sample-derived measures.

"Measuring", "measurement", "detection" and "detecting" mean assessing the presence, absence, quantity or amount (which can be an effective amount) of either a given substance within a clinical or subject-derived sample, including qualitative or quantitative concentration levels of such substances, or otherwise evaluating the values or categorization of a subject's clinical parameters.

"Altered", "an increase" or "a decrease" refers to a detectable change or difference between the measured biomarker and the reference value from a reasonably comparable state, profile, measurement, or the like. One skilled in the art should be able to determine a reasonable measurable change. Such changes may be all or none. They may be incremental and need not to be linear. They may be by orders of magnitude. A change may be an increase or decrease by 1%, 5%, 10%, 20%,30%, 40%, 50%, 60%, 70%, 80%, 90%, 95%, 99%, 100%, or more, or any value in between 0% and 100%. Alternatively, the change may be 1-fold, 1.5- fold, 2-fold, 3-fold, 4-fold, 5-fold or more, or any values in between 1-fold and five-fold. The change may be statistically significant with a p value of 0.1, 0.05, 0.001, or 0.0001.

A "female subject" can be one who has not been previously diagnosed or identified as having breast tumor. A female subject can be a healthy subject who is classified as low risk for developing a breast cancer condition. Alternatively, a female subject can be one who has a risk of developing breast cancer or tumor. A risk factor is anything that affects the subject's chance of getting a disease such as breast cancer or tumor.

More exactly, a female subject can be one who has been previously diagnosed with or identified as suffering from or having breast cancer, and optionally, but need not have already undergone treatment for the breast cancer. A female subject can also be one who is not suffering from breast cancer. A female subject can also be one who has been diagnosed with or identified as suffering from breast cancer, but who shows improvements in the disease (such as, for example, a decrease in tumor size) as a result of receiving one or more treatments for breast cancer. Alternatively, a female subject can also be one who has not been previously diagnosed or identified as having breast cancer. For example, a female subject can be one who exhibits one or more risk factors for breast cancer, or a female subject who does not exhibit risk factors for breast cancer, or a subject who is asymptomatic for breast cancer. A female subject can also be one who is suffering from or at risk of developing breast cancer.

By "asymptomatic" female subject it is intended a female subject showing no breast cancer symptoms. This definition may comprise the detection of an early breast cancer in a female subject that is mammography-negative. One advantage of the invention is the detection or diagnosis of breast cancer at an early stage.

However, the goal of the present invention is not limited to the detection of breast cancer in a woman or female subject having a negative mammography but also the detection of breast cancer independently from mammography and preferably before mammography becomes positive. This is also true for the screening test or kit according to the invention. The kit is to detect a potential breast cancer in a woman who do not have any symptom of the disease. Preferably the female subject has not been previously diagnosed or identified as having a breast cancer, however said female subject is at risk of developing breast cancer. Thus, the asymptomatic subject in that case is a female subject carrying a breast cancer but who experiences no symptoms.

A "biological sample" in the context of the present invention is a biological sample isolated from a female subject and can include, by way of example and not limitation, whole blood, serum, plasma, blood cells, peripheral blood mononuclear cells, endothelial cells, circulating tumor cells, tissue biopsies, lymphatic fluid, ascites fluid, interstitial fluid, bone marrow, cerebrospinal fluid (CSF), saliva, mucous, sputum, sweat, urine, or any other secretion, excretion, or other bodily fluids. In some embodiments, the sample refers to peripheral blood mononuclear cells or blood cells.

"Peripheral blood mononuclear cell" (PBMC) refers to any cell present in the blood having a round nucleus. This fraction is conventionally isolated by centrifuging whole blood in a liquid density gradient. It contains mainly lymphocytes and monocytes while excluding red blood cells and granulocytes (eosinophils, basophils, and neutrophils). Rare cells with a round nucleus such as progenitor endothelial cells or circulating tumor cells could also be present in this fraction.

The term "primer" refers to a strand of nucleic acid having usually between 8 and 25 nucleotides that serves as a starting point for DNA replication.

The terms "probe" and "hydrolysis probe" refer to a short strand of nucleic acid designed to hybridize to a region within the amplicon and is dual labeled with a reporter dye and a quenching dye. The close proximity of the quencher suppresses the fluorescence of the reporter dye. The probe relies on the 5'-3' exonuclease activity of Taq polymerase, which degrades a hybridized non-extendible DNA probe during the extension step of the polymerase chain reaction (PCR). Once the Taq polymerase has degraded the probe, the fluorescence of the reporter increases at a rate that is proportional to the amount of template present.

The term "gene expression" means the production of a protein or a functional mRNA from its gene.

The terms "signature", "classifier", "model" and "predictor" are used interchangeably. They refer to an algorithm that discriminates between disease states with a predetermined level of statistical significance. A two-class classifier is an algorithm that uses data points from measurements from a sample and classifies the data into one of two groups. In certain embodiments, the data used in the classifier is the relative expression of nucleic acids or proteins in a biological sample. Protein or nucleic acid expression levels in a subject can be compared to levels in patients previously diagnosed as disease free or with a specified condition.

A "reference or baseline level/value" as used herein can be used interchangeably and is meant to be relative to a number or value derived from population studies, including without limitation, such subjects having similar age range, disease status (e.g., stage), subjects in the same or similar ethnic group, or relative to the starting sample of a subject undergoing treatment for cancer. Such reference values can be derived from statistical analyses and/or risk prediction data of populations obtained from mathematical algorithms and computed indices of breast cancer. Reference indices can also be constructed and used utilizing algorithms and other methods of statistical and structural classification.

In some embodiments of the present invention, the reference or baseline value is the expression level of a particular biomarker of interest in a control sample derived from one or more healthy female subjects or subjects who have not been diagnosed with any breast cancer.

In some embodiments of the present invention, the reference or baseline value is the expression level of a particular biomarker of interest in a sample obtained from the same subject prior to any cancer treatment. In other embodiments of the present invention, the reference or baseline value is the expression level of a particular biomarker of interest in a sample obtained from the same subject during a cancer treatment. Alternatively, the reference or baseline value is a prior measurement of the expression level of a particular gene of interest in a previously obtained sample from the same subject or from a subject having similar age range, disease status (e.g., stage) to the tested subject.

The term "ruling out" as used herein is meant that the subject is selected not to receive a treatment protocol.

The term "ruling in" as used herein is meant that the subject is selected to receive a treatment protocol.

The term "normalization" or "normalizer" as used herein refers to the expression of a differential value in terms of a standard value to adjust for effects which arise from technical variation due to sample handling, sample preparation and mass spectrometry measurement rather than biological variation of protein concentration in a sample. For example, when measuring the expression of a differentially expressed protein (nucleic acid), the absolute value for the expression of the protein (nucleic acid) can be expressed in terms of an absolute value for the expression of a standard protein (nucleic acid) that is substantially constant in expression. This prevents the technical variation of sample preparation and PCR measurement from impeding the measurement of protein (nucleic acid) concentration levels in the sample.

The term "score" or "scoring" refers to calculating a probability likelihood (or a probability value) by the model (e.g., a logistic regression model) for a sample. For the present invention, values closer to 1.0 are used to represent the likelihood that a sample is derived from a patient with a breast cancer condition, values closer to 0.0 represent the likelihood that a sample is derived from a patient without a breast cancer condition.

A "pre-determined score" refers to a probability threshold that has been determined during the modeling/training phase by, for instance, logistic regression and receiver operating characteristic ROC analysis, and that defines the likelihood of breast tumor and/or diagnosis of breast tumor. A skilled artisan can readily determine such score according to any methods available in the art.

"Transcriptomic (mRNA) markers" are specific mRNA molecules and their quantity present in a given cell or tissue that correlate with a particular cell population or tissue type, state of activation, differentiation, or function. In disease conditions, transcriptomic (mRNA) markers may have diagnostic, prognostic or predictive value.

"Cell surface markers" specific for the immune response elicited by breast cancer at its different stages are proteins, carbohydrates, lipids or any molecular moieties, and their quantity that are detectable at the cell surface by analytical tools, including antibodies, peptides, nucleic acids (aptamers), small molecules, or chemicals, that correlate with a particular cell population or tissue type, different states of activation, differentiation, and function. In disease conditions, cell surface markers may have diagnostic, prognostic or predictive value.

One object of the invention is to provide a breast cancer detection method for a female subject, comprising:

(a) measuring in a biologic sample obtained from said female subject the expression level of transcriptomic (mRNA) markers of a first panel comprising the combination of SOX4, TNFSF10, CD3G, and NR3C2 and cell surface biomarkers specific for the immune response elicited by breast cancer at its different stages of a second panel comprising the combination of CD11b, CD62L, CD86, CD117, CD144, CD177, CD202b;
(b) calculating a probability score based on the measurement of step (a); and
(c) ruling out breast cancer for said female subject if the score in step (b) is lower than a predetermined score; or
(d) ruling in the likelihood of breast cancer for said female subject if the score in step (b) is higher than a pre-determined score.

Preferably, the cell surface biomarkers specific for the immune response elicited by breast cancer at its different stages of the second panel further comprises at least one, at least 2, 3, 4, 5, 6 or 7 protein biomarkers selected from the group comprising FcεRI, HLA-DR, CD69, CD101, CD163, CD170, and CD274 or a combination thereof.

Even more preferably, the cell surface biomarkers specific for the immune response elicited by breast cancer at its different stages of the second panel further comprises at least one, at least 2, 3, 4, 5, 6, 7 or 8 protein biomarkers selected from the group comprising CD3, CD14, CD16, CD15, CD19, CD20, CD56, and CD66b or a combination thereof.

According to another embodiment, the transcriptomic (mRNA) markers of said first panel further comprises at least one, at least 2, 3, 4, 5, 6, 7, 8 or 9 transcript markers selected from the group comprising FCER1A, GZMH, KLF12, HLA-DOA, CX3CR1, HMGB2, LY9, S1PR1, and KLRB1 or a combination thereof.

Preferably, the transcriptomic (mRNA) markers of said first panel further comprises at least one, at least 2, 3, 4, 5, 6, 7, 8, 9, 10, 11, 12, 13, 14 or 15 transcript markers selected from the group comprising TGFBR3, CCL20, CCR3, FN1, ACKR3, IL10, CXCL10, C3, MKI67, HBEGF, C9orf47, CD40, EREG, CXCL9, and SERPINE1 or a combination thereof.

Different biomarker combinations are necessary to define the relevant populations for example: Polymorphonuclear granulocytes: FSC/SSC gating for granulocytes; CD14-; CD15+, CD66b+; CD11b++, CD16+.

Classical monocytes: FSC/SSC gating for monocytes; CD11b+; CD14++, CD16-. Intermediate monocytes: FSC/SSC gating for monocytes; CD11b+; CD14++, CD16+. Non-classical monocytes: FSC/SSC gating for monocytes; CD11b+; CD14-, CD16+.

Lineage markers are necessary to define leukocyte population of interest, in which the above biomarkers are selected from the list comprising or consisting of CD11b, CD62L, CD86, CD117, CD144, CD177, CD202b will discriminate pBC (primary breast cancer), mBC (metastatic or recurrent breast cancer), patients vs healthy donor (HD).

The probability score can be calculated according to any method known in the art. For example, the probability score is calculated from a logistic regression prediction model applied to the measurement by an algorithm.

In some embodiments, the likelihood of breast cancer is also determined by the sensitivity, specificity, negative predictive value (NPV) or positive predictive value (PPV) associated with the score.

According to a further embodiment of the invention, the detection is an early detection or a detection of follow-up breast cancers at any stage of the disease.

It is another object of the invention to provide for a breast cancer classifying or stratifying prognostic method for classifying/stratifying whether a female subject is more likely to develop breast cancer comprising:

a) measuring in a biologic sample obtained from said female subject the expression level of transcriptomic (mRNA) markers of a first panel comprising the combination of SOX4, TNFSF10, CD3G, and NR3C2, and cell surface biomarkers specific for the immune response elicited by breast cancer at its different stages of a second panel comprising the combination of CD11b, CD62L, CD86, CD117, CD144, CD177, CD202b;

(b) comparing the amount measured in step (a) to a reference value; and

(c) classifying the female subject as more likely to have progredient breast cancer when an increase or a decrease in the amount of each transcriptomic (mRNA) markers of the first panel and in the amount of at least one protein biomarker of the second panel relative to a reference value is detected in step (b).

Preferably, the cell surface biomarkers specific for the immune response elicited by breast cancer at its different stages of the second panel further comprises at least one, at least 2, 3, 4 , 5, 6 or 7 protein biomarkers selected from the group comprising FcεRI, HLA-DR, CD69, CD101, CD163, CD170, and CD274 or a combination thereof.

Even more preferably, the cell surface biomarkers specific for the immune response elicited by breast cancer at its different stages of the second panel further comprises at least one, at least 2, 3, 4, 5, 6, 7 or 8 protein biomarkers selected from the group comprising CD3, CD14, CD16, CD15, CD19, CD20, CD56, and CD66b or a combination thereof.

According to another embodiment, the transcriptomic (mRNA) markers of said first panel further comprises at least one, at least 2, 3, 4 , 5, 6, 7, 8 or 9 transcript markers selected from the group comprising FCER1A, GZMH, KLF12, HLA- DOA, CX3CR1, HMGB2, LY9, S1PR1, and KLRB1or a combination thereof.

Preferably, the transcriptomic (mRNA) markers of said first panel further comprises at least one, at least 2, 3, 4, 5, 6, 7, 8, 9, 10, 11, 12, 13, 14 or 15 transcript markers selected from the group comprising TGFBR3, CCL20, CCR3, FN1, ACKR3, IL10, CXCL10, C3, MKI67, HBEGF, C9orf47, CD40, EREG, CXCL9, and SERPINE1or a combination thereof.

In an embodiment, when breast cancer is ruled out, the female subject does not receive a treatment protocol.

In another embodiment, when breast cancer is ruled in, the female subject is to receive a treatment protocol. Preferably, said treatment protocol is a biopsy, a surgery, a chemotherapy, a radiotherapy, or any combination thereof.

In the breast cancer detection method or the breast cancer classifying or stratifying prognostic method of the invention, the biologic sample is selected from the group consisting of peripheral blood mononuclear cells, blood cells, whole blood, serum, plasma, circulating tumor cells, lymphatic fluid, ascites fluid, interstitial fluid, bone marrow, cerebrospinal fluid (CSF), saliva, mucous, sputum, and urine and preferably selected from the group consisting of peripheral blood mononuclear cells, blood cells, whole blood, serum, plasma, circulating tumor cells, lymphatic fluid, bone marrow and cerebrospinal fluid (CSF).

In the breast cancer detection method or the breast cancer classifying or stratifying prognostic method of the invention, usually the breast cancer is a carcinoma.

According to an embodiment of the breast cancer detection method or the breast cancer classifying or stratifying prognostic method of the invention, the likelihood of breast cancer is further determined by the sensitivity, specificity, negative predictive value (NPV) or positive predictive value (PPV) associated with the score.

According to another embodiment of the breast cancer detection method or the breast cancer classifying or stratifying prognostic method of the invention, the female subject is at risk of developing primary or recurrent breast cancer.

It is yet another object of the invention to provide a method for treating breast cancer in a female subject, the method comprising:

a) measuring in a biologic sample obtained from said female subject the expression level of transcriptomic (mRNA) markers of a first panel comprising the combination of SOX4, TNFSF10, CD3G, and NR3C2, and cell surface biomarkers specific for the immune response elicited by breast cancer at its different stages of a second panel comprising the combination of CD11b, CD62L, CD86, CD117, CD144, CD177, CD202b;

(b) comparing the amount measured in step (a) to a reference value; wherein an increase or a decrease in the amount of each transcriptomic (mRNA) markers of the first panel and in the amount of at least one protein biomarker of the second panel relative to a reference value indicates that the female subject suffers from breast cancer;

(c) administering to said female subject at least one breast cancer-modulating agent when the subject suffers from breast cancer identified by step (b).

In particular, the reference value comprises an index value, a value derived from one or more breast cancer risk prediction algorithms or computed indices, a value derived from a female subject not suffering from breast cancer, or a value derived from a female subject diagnosed with or identified as suffering from breast cancer.

The female subject comprises one who has been previously diagnosed as having breast cancer, one who has not been previously diagnosed as having breast cancer, or one who is asymptomatic for the breast cancer.

Advantageously, the female subject classified in step (b) can be treated by one of the following therapeutic modalities, or combinations thereof, referred herein as at least one breast cancer-modulating agent.

**Surgical therapy:** it consists in the removal of the tumor and some surrounding healthy tissue during an operation. Surgery is also used to examine the nearby axillary lymph nodes to determine disease spreading. Surgery can be performed as lumpectomy, the removal of the tumor and a small cancer-free margin of healthy tissue around the tumor. Mastectomy, the removal of the entire breast.

**Radiation therapy:** it consists in the use of high-energy x-rays or other particles to destroy cancer cells and lower the risk of recurrence in the breast. Radiotherapy can be applied as external-beam therapy, given from a machine outside the body; as intra-operatively, when radiation treatment is given using a probe in the operating room, as brachytherapy, when given by placing radioactive sources into the tumor.

**Chemotherapy:** it consists in the use of drugs to kill cancer cells or keeping the cancer cells from dividing and growing. It may be given before surgery to shrink a large tumor, to make surgery easier, and/or to reduce the risk of recurrence. This is called neoadjuvant chemotherapy. Chemotherapy can be given after surgery to reduce the risk of recurrences. It can be given once a week, once every 2 weeks, once every 3 weeks, or once every 4 weeks. There are many types of chemotherapy used to treat breast cancer. Exemplary breast cancer-chemotherapy agents include, but are not limited to: Docetaxel (Taxotere); Paclitaxel (Taxol); Doxorubicin; Epirubicin (Ellence); Pegylated liposomal doxorubicin (Doxil); Capecitabine (Xeloda); Carboplatin (available as a generic drug); Cisplatin (available as a generic drug); Cyclophosphamide (available as a generic drug); Eribulin (Halaven); Fluorouracil (5-FU); Gemcitabine (Gemzar); Ixabepilone (Ixempra); Methotrexate (Rheumatrex, Trexall); Protein-bound paclitaxel (Abraxane); Vinorelbine (Navelbine).

**Hormonal therapy:** it consists in the administration of agents suppressing hormonal signalling. It is an effective treatment for most tumors expressing oestrogen and progesterone receptors ($ER^+ PR^+$). It can be given before surgery, typically for at least 3 to 6 months, and or after surgery and continued for 5 to 10 years. Blocking the hormones can help prevent a cancer recurrence and death from breast cancer when used either alone or after chemotherapy. Exemplary hormonal therapy agents include, but are not limited to: Tamoxifen (Novaldex) blocks oestrogen from binding to its receptor; Aromatase inhibitors, including anastrozole (Arimidex), exemestane (Aromasin), and letrozole (Femara) inhibit synthesis of estrogen; Ovarian suppression using gonadotropin or luteinizing releasing hormone (GnRH or LHRH) agonist to stop the ovaries from making oestrogen such as Goserelin (Zoladex) and leuprolide (Eligard, Lupron); Ovarian ablation, the surgical removal of the ovaries.

**HER2-targeted therapy:** it is used to treat HER2 breast cancers. Many HER2 inhibitors are available including, but no limited to: Trastuzumab (Herceptin); Pertuzumab (Perjeta), Neratinib (Nerlynx), Ado-trastuzumab emtansine or T-DM1 (Kadcyla) are antibodies given intravenously. Lapatinib (Tykerb), Tucatinib (Tukysa) are orally available HER2 tyrosine kinase inhibitors.

Other targeted drugs used in breast cancer therapy include: Olaparib (Lynparza), PARP inhibitor; abemaciclib (Verzenio), palbociclib (Ibrance), and ribociclib (Kisqali), CDK4/6 inhibitors; PI3K inhibitor Alpelisib (Piqray); Sacituzumab govitecan-hziy (Trodelvy); Entrectinib (Rozyltrek) and larotrectinib (Vitrakvi); Talazoparib (Talzenna).

**Immunotherapy:** it is used to stimulate the immune system to attack cancer cells, using drugs called immune checkpoint inhibitors. The following drugs are used for recurrent, advanced or metastatic breast cancer : Pembrolizumab (Keytruda), PD-L1 inhibitor; Ipilimumab (Yervoy), CTLA-4 inhibitor; Dostarlimab (Jemperli), PD-1 inhibitor.

Also provided is a breast cancer detection kit for use in the detection, stratification and/or monitoring of a likelihood of breast cancer in an asymptomatic female subject from a peripheral blood sample, said kit comprising:

- at least one probe for measuring the expression level of transcriptomic (mRNA) markers of a first panel of four genes comprising the combination of SOX4, TNFSF10, CD3G, and NR3C2; and
- at least one probe and/or specific detection reagent for measuring the expression level of seven cell surface biomarkers specific for the immune response elicited by breast cancer at its different stages of a second panel comprising the combination of CD11b, CD62L, CD86, CD117, CD144, CD177, and CD202b.

Preferably at least one probe and/or specific detection reagent for measuring one or more cell surface biomarker of the second panel comprises FcεRI, HLA-DR, CD69, CD101, CD163, CD170, and CD274 or a combination thereof.

More preferably, the at least one probe and/or specific detection reagent for measuring one or more cell surface biomarker of the second panel comprises CD3, CD14, CD16, CD15, CD19, CD20, CD56, and CD66b or a combination thereof.

According to another embodiment, the at least one probe for measuring one or more transcriptomic (mRNA) marker of the first panel comprises FCER1A, GZMH, KLF12, HLA-DOA, CX3CR1, HMGB2, LY9, S1PR1, and KLRB1or a combination thereof.

Preferably, the at least one probe for measuring one or more transcriptomic (mRNA) marker of the first panel comprises TGFBR3, CCL20, CCR3, FN1, ACKR3, IL10, CXCL10, C3, MKI67, HBEGF, C9orf47, CD40, EREG, CXCL9, and SERPINE1 or a combination thereof.

Advantageously, the kit for use of the invention further comprises primer pairs specific for one or more housekeeping genes selected from the list comprising TBP, SDHA, ACTBIPO8, HuPO, BA, CYC, GAPDH, PGK, B2M, GAPDH.

In addition, said kit for use further comprises one or more probes, reference samples for performing measurement quality controls.

In practice, two scores can be calculated based on the results obtained from the kit of the invention. One score indicative of primary breast cancer and another one for metastatic breast cancer.

Ideally, the kit for use of the invention further comprises one or more plastic container and reagents for performing test reactions and optionally instructions for use.

The kit for use can contain reagents that specifically bind to proteins in the panels described, herein. These reagents can include antibodies.

In case breast cancer is ruled in, the female subject is to receive a treatment protocol. Preferably, said treatment protocol is a biopsy, a surgery, a chemotherapy, a radiotherapy, or any combination thereof.

The actual measurement of levels of the biomarkers of the invention namely the transcriptomic (mRNA) markers of panel 1 and the cell surface biomarker of panel 2, can be determined at the nucleic acid or protein level using any method known in the art. For example, at the nucleic acid level, the biomarkers can be measured by extracting ribonucleic acids from the sample and performing any type of quantitative PCR on the reverse-transcribed nucleic acids. Another way to detect the biomarkers can also be by a whole transcriptome analysis based on high-throughput sequencing methodologies, e.g., RNA-seq, or on microarray technology, e.g., Affymetrix arrays.

By way of example, other methods that can be used for measuring the biomarker may involve any other method of quantification known in the art of nucleic acids, such as but not limited to amplification of specific sequences, oligonucleotide probes, hybridization of target genes with complementary probes, fragmentation by restriction endonucleases and study of the resulting fragments (polymorphisms), pulsed field gels techniques, isothermic multiple-displacement amplification, rolling circle amplification or replication, immuno-PCR, among others known to those skilled in the art.

By using information provided by database entries for the biomarker sequences, biomarker expression levels can be detected and measured using techniques well known to one of ordinary skill in the art. For example, biomarker sequences within the sequence database entries, or within the sequences disclosed herein, can be used to construct probes and primers for detecting biomarker mRNA sequences in methods which specifically, and, preferably, quantitatively amplify specific nucleic acid sequences such as reverse-transcription based real-time polymerase chain reaction (RT-qPCR).

Levels of biomarkers can also be determined at the protein level, e.g., by measuring the levels of peptides encoded by the gene products described herein, or activities thereof. Such methods are well known in the art and include, e.g., immunoassays based on antibodies to proteins encoded by the genes, aptamers or molecular imprints, cell surface staining and flow cytometry analyses. Alternatively, a suitable method can be selected to determine the activity of proteins encoded by the biomarker genes according to the activity of each protein analyzed.

The biomarker proteins, polypeptides, mutations, and polymorphisms thereof can be detected in any suitable manner, but is typically detected by contacting a sample from the subject with an antibody which binds the biomarker protein, polypeptide, mutation, or polymorphism and then detecting the presence or absence of a reaction product. The antibody may be monoclonal, polyclonal, chimeric, or a fragment of the foregoing, and the step of detecting the reaction product may be carried out with any suitable immunoassay. The sample from the subject is typically a biological sample as described above, and may be the same sample used to conduct the method described above.

Those skilled in the art will be familiar with numerous specific immunoassay and nucleic acid amplification assay formats and variations thereof which may be useful for carrying out the embodiments of the invention disclosed herein.

Preferably, expression levels of the biomarkers of the present invention are detected by RT-qPCR, and in particular by real-time PCR, as described further herein.

In general, total RNA can be isolated from the target sample, such as peripheral blood or PBMC, using any isolation procedure. This RNA can then be used to generate first strand copy DNA (cDNA) using any procedure, for example, using random primers, oligo-dT primers or random-oligo-dT primers which are oligo-dT primers coupled on the 3'-end to short stretches of specific sequence covering all possible combinations. The cDNA can then be used as a template in quantitative PCR.

In real-time PCR quantification of PCR products relies, for example, on increases in fluorescence, released at each amplification cycle of the reaction, for example, by a probe that hybidizes to a portion of the amplification product. Fluorescence approaches used in real-time quantitative PCR are typically based on a fluorescent reporter dye such as FAM, fluorescein, HEX, TET, etc. and a quencher such as TAMRA, DABSYL, Black Hole, etc. When the quencher is separated from the probe during the extension phase of PCR, the fluorescence of the reporter can be measured. Systems like Universal ProbeLibrary, Molecular Beacons, Taqman Probes, Scorpion Primers or Sunrise Primers and others use this approach to perform real-time quantitative PCR. Alternatively, fluorescence can be measured from DNA-intercalating fluorochromes such as Sybr Green.

The abundance of target RNA molecules can be performed by real-time PCR in a relative or absolute manner. Relative methods can be based on the threshold cycle determination (Ct) or, in the case of the Roche's PCR instruments, the crossing point (Cp). Relative RNA molecule abundance is then calculated by the delta Ct (delta Cp) method by subtracting Ct (Cp) value of one or more housekeeping genes. Alternatively, absolute measurement can be performed by determining the copy number of the target RNA molecule by the mean of standard curves.

The application of logistic regression to biological problems is routine in the art. Various statistical analysis software, can be used for building logistic regression models. Fitted logistic regression models are tested by asking whether the model can correctly predict the clinical outcome using patient data other than that with which the logistic regression model was fitted but having a known clinical outcome. After training, the model output from 0 (control) to 1 (cancer) can be calculated in blind fashion by the average error of all N predictions (a validation group). Based on the output values, the receiver operating characteristic (ROC) curve can be built to calculate the outcome of clinical prediction: specificity and sensitivity of breast cancer detection. They are statistical measures of the performance of a binary classification test. Sensitivity measures the proportion of actual positives which are correctly identified as such (e.g., the percentage of sick women who are correctly identified as having the condition). Specificity measures the proportion of negatives which are correctly identified (e.g., the percentage of healthy women who are correctly identified as not having the condition). A perfect predictor would be described as 100% sensitive (i.e., predicting all women from the sick group as sick) and 100% specific (i.e., not predicting anyone from the healthy group as sick). However, any predictor will possess a minimum error bound.

One embodiment of the present disclosure is a predictive model comprising a combination/profile of peripheral blood mononuclear cell biomarkers detecting breast tumors preferably with sensitivity equal or above to 60%, preferably equal or above 70%, more preferably equal or above 80% and even more preferably equal or above 85% and specificity equal or above 84%, preferably equal or above 85%.

The term "sensitivity of a test" refers to the probability that a test result will be positive when the disease is present in the patient (true positive rate). This is derived from the number of patients with the disease who have a positive test result (true positive) divided by the total number of patients with the disease, including those with true positive results and those patients with the disease who have a negative result, i.e., false negative.

The term "specificity of a test" refers to the probability that a test result will be negative when the disease is not present in the patient (true negative rate). This is derived from the number of patients without the disease who have a negative test result (true negative) divided by all patients without the disease, including those with a true negative result and those patients without the disease who have a positive test result, e.g. false positive. While the sensitivity, specificity, true or false positive rate, and true or false negative rate of a test provide an indication of a test's performance, e.g. relative to other tests, to make a clinical decision for an individual patient based on the test's result, the clinician requires performance parameters of the test with respect to a given population.

The term "positive predictive value" (PPV) refers to the probability that a positive result correctly identifies a patient who has the disease, which is the number of true positives divided by the sum of true positives and false positives.

The term "negative predictive value" or "NPV" refers to the probability that a negative test correctly identifies a patient without the disease, which is the number of true negatives divided by the sum of true negatives and false negatives. Like the PPV, it also is inherently impacted by the prevalence of the disease and pre-test probability of the population intended to be tested. A positive result from a test with a sufficient PPV can be used to rule in the disease for a patient, while a negative result from a test with a sufficient NPV can be used to rule out the disease, if the disease prevalence for the given population, of which the patient can be considered a part, is known.

A "Receiver Operating Characteristics (ROC) curve" as used herein refers to a plot of the true positive rate (sensitivity) against the false positive rate (specificity) for a binary classifier system as its discrimination threshold is varied. A ROC curve can be represented equivalently by plotting the fraction of true positives out of the positives (TPR=true positive rate) versus the fraction of false positives out of the negatives (FPR=false positive rate). Each point on the ROC curve represents a sensitivity/specificity pair corresponding to a particular decision threshold.

AUC (Area Under the Curve) represents the area under the ROC curve. The AUC is an overall indication of the diagnostic accuracy of 1) a biomarker or a panel of biomarkers and 2) a ROC curve. AUC is determined by the "trapezoidal rule." For a given curve, the data points are connected by straight line segments, perpendiculars are erected from the abscissa to each data point, and the sum of the areas of the triangles and trapezoids so constructed is computed. In certain embodiments of the methods provided herein, a biomarker protein has an AUC in the range of about 0.75 to 1.0. In certain of these embodiments, the AUC is in the range of about 0.8 to 0.8, 0.9 to 0.95, or 0.95 to 1.0.

The methods provided herein are minimally invasive and pose little or no risk of adverse effects. As such, they may be used to diagnose, monitor and provide clinical management of subjects who do not exhibit any symptoms of a breast cancer condition and subjects classified as low risk for developing a breast cancer condition. Similarly, the methods disclosed herein may be used as a strictly precautionary measure to diagnose healthy subjects who are classified as low risk for developing a breast cancer condition.

Applicants reported that the proangiogenic factor Placenta Growth Factor (PlGF) programs CD11[+] myelomonocytes in breast cancer (BC) patients to become pro-angiogenic and to promote tumor growth. Applicants could also show that CD11[+] myelomonocytes present in the blood of BC patients are proangiogenic. Applicants were able experimentally to reprogram the angiogenic activity of CD11[+] myelomonocytes in vitro with PlGF. This observation demonstrated that circulating myelomonocytes are primed to acquire new functional properties in BC patients. Taken together these results show that cancer cells can program proangiogenic activity in CD11b[+] myelomonocytes during differentiation of their progenitor cells in a PlGF-dependent manner [10].

In a later study, Applicants demonstrated that the angiogenic activity of BC patients' blood monocytes previously reported can be reverted by the combined use of systems modeling and experimental approaches. Specifically, these results highlighted the crosstalk between angiogenic and inflammatory signaling pathways controlling the pro-angiogenic and tumor promoting activity of CD11b[+] cells [11].

Using the 4T1 experimental BC model, Applicants observed that that mobilization of CD11b[+]c-Kit[+]Ly6G[high]-Ly6C[low]Grl[+] myeloid cells in response to radiotherapy promotes metastasis in a preclinical model of BC. Mobilization is mediated by the expression of Kit ligand (KitL) by hypoxic tumor cells. Inhibition of KitL/Kit suppressed CD11b[+]c-Kit[+]Ly6G[high]ly6C[low]Gr1[+] myeloid cell mobilization and metastasis formation. Depletion of CD11b[+]c-Kit[+] cells also suppressed metastasis formation. From these results the Applicants concluded that mobilized CD11b[+]c-Kit[+] in the blood are reporter and mediator of BC metastasis after adjuvant radiotherapy. This work defined KitL/c-Kit as a previously unidentified axis critically involved in promoting metastasis of 4T1 tumors growing in pre-irradiated mammary tissue. Pharmacological inhibition of this axis represents a potential therapeutic strategy to prevent metastasis in breast cancer patients with local relapses after radiotherapy [12].

Using the same model, Applicants performed gene expression analyses on Kit[+]peripheral blood cells, obtained from tumor free and tumor bearing mice that were locally irradiated on the mammary region with 20 Gy single dose prior tumor cell injection (to mimic post radiation relapse). It was observed that Kit[+] cells obtained from pre-irradiated tumor bearing mice, but not from the other mice, had a different gene expression signature: 55 gene were significantly expressed differently. This work demonstrated that circulating CD11b[+]c-Kit[+] cells have a plastic transcriptomic profile influenced by the presence of the growing after adjuvant-like radiotherapy.

In a case-control clinical study, Applicants observed that metastatic BC (mBC) patients have elevated levels of circulating KIT[+]CD11b[+] cells (**Figure 1**) and IL-10 and that the anti-VEGF antibody bevacizumab (Avastin) specifically reverses them to values observed in clinically tumor free patients. Applicants also analyzed the transcriptome of CD11b[+] myeloid cells derived from four mBC patients and 4 healthy donors (HD). Circulating CD11b[+] cells in mBC patients have a distinctive gene expression profile compared to HD. CD11b[+] cells from mBC patients expressed genes of M2 monocytes' activation state (i.e. IL-10 and CCL20) (**Figure 2**). Gene ontology analysis of the differentially expressed genes reveled that expression of immune response genes was reduced in mBC, consistent with a M2 tumor-induced CD11b[+] cells polarization. The expression of genes of M2 polarization identified by gene profiling (i.e. CD163, ARG1, IL10) were confirmed by RT-qPCR on the 20 patients of the study. Treatments with bevacizumab reversed their profile to the level observed in HD. This case-control study provided evidence of systemic immunomodulatory effects of bevacizumab and identified circulating KIT[+]CD11b[+] cells and IL-10 as candidate biomarkers of mBC and bevacizumab activity [9].

Applicants performed a further clinical study to monitoring the effects of adjuvant breast cancer radiotherapy on the phenotype of blood circulating immune/inflammatory cells by flow cytometry coupled with T-distributed Stochastic Neighbor Embedding (t-SNE) unsupervised analysis of global cell surface expression data (**Figure 3**). With this approach they identified non-standard populations that were not identified with conventional approaches, including cells negative for all tested markers or expressing unanticipated marker combinations (**Figure 4**). Applicants demonstrated that patients with newly diagnosed breast cancer had a significantly elevated frequency of cKIT[+]CD11b[+] cells and a decrease number of CD163[+]CD11b[+] cells (**Figure 5**). Radiotherapy further increased the frequency of cKIT[+]CD11b[+] cells. This study demonstrates the value of unsupervised analysis of complex flow cytometry data to unravel new cell populations of potential clinical relevance.

The present invention is based on the quantitative detection, of multiple transcripts (mRNA) and cell surface protein (biomarkers) in peripheral blood leukocytes, their association and analysis.

Biomarkers are modulated by the presence of a breast cancer at any stage.

Molecular techniques (including but not limited to, Polymerase Chain Reaction - PCR, RNASeq, hybridization) are used to detect mRNA; fluorescence antibodies and flow cytometry (FACS) or nucleic acid tagged antibodies and molecular techniques (including but not limited to, PCR, RNASeq, hybridization) are used to detect proteins.

Results are analyzed by an algorithm calculating a score to identify women with breast cancer at any stage, from healthy women without breast cancer (diagnosis) or to stratify patients with relapsing breast cancer from patients with non-relapsing breast cancer after initial therapy (prognostic)

Patients with a positive result indicating the presence of a breast cancer or a breast cancer relapse will be subjected to further diagnostic validation steps and treated accordingly.

The invention requires minimally invasive sample collection (peripheral blood) processes than tumor biopsy, which is considered nowadays as the gold-standard and required for definitive diagnosis.

The sensitivity of Applicant's approach is better compared to tumor-derived markers, as is it is based on a natural amplification reaction of the immune system elicited by the presence of a minute number of cells, resulting in the generation of a measurable signal revealed by the detection kit according to the invention. The present invention leads to a more sensitive detection compared to detection via signals directly released by cancer cells. The cancer can be detected earlier thereby increasing likelihood of a successful treatment, improved survival and quality of life.

Besides, the invention can be combined with existing methods of detection in particular with those based on tumor-derived material.

The list of the transcriptomic biomarkers (mRNA) is given in Table 1, Table 2 and Table 3, respectively.

Table 1. First priority transcriptome biomarkers

|  | Gene symbol | Protein name | GeneI D | Gene ID (Ensembl) |
|---|---|---|---|---|
| 1 | SOX4 | SRY-Box Transcription Factor 4 | 6659 | ENSG00000124766 |
| 2 | TNFSF10 | TRAIL | 8743 | ENSG00000121858 |
| 3 | CD3G | T-Cell Surface Glycoprotein CD3 Gamma Chain | 917 | ENSG00000160654 |
| 43 | NR3C2 | Aldosterone Receptor | 4306 | ENSG00000151623 |

Table 2. Second priority transcriptome biomarkers

|  | Gene symbol | Protein name | GeneI D | Gene ID (Ensembl) |
|---|---|---|---|---|
| 5 | FCER1A | Immunoglobulin E Receptor, High-Affinity, Of Mast Cells | 2205 | ENSG00000179639 |
| 6 | GZMH | Granzyme H | 2999 | ENSG00000100450 |
| 7 | KLF12 | Kruppel Like Factor 12 | 11278 | ENSG00000118922 |
| 8 | HLA-DOA | Major Histocompatibility Complex, Class II, DO Alpha | 3111 | ENSG00000204252 |
| 9 | CX3CR1 | Fractalkine Receptor | 1524 | ENSG00000168329 |
| 10 | HMGB2 | High Mobility Group Box 2 | 3148 | ENSG00000164104 |
| 11 | LY9 | T-Lymphocyte Surface Antigen Ly-9 | 4063 | ENSG00000122224 |
| 12 | S1PR1 | Sphingosine-1-Phosphate Receptor 1 | 1901 | ENSG00000170989 |
| 13 | KLRB1 | Natural Killer Cell Surface Protein P1A | 3820 | ENSG00000111796 |

Table 3. Third priority transcriptome biomarkers

|  | Gene symbol | Protein name | GeneI D | Gene ID (Ensembl) |
|---|---|---|---|---|
| 14 | TGFBR3 | Transforming Growth Factor Beta Receptor 3 | 7049 | ENSG00000069702 |
| 15 | CCL20 | MIP3alpha | 6364 | ENSG00000115009 |
| 16 | CCR3 | Eosinophil Eotaxin Receptor | 1232 | ENSG00000183625 |
| 17 | FNI | Fibronectin 1 | 2335 | ENSG00000115414 |
| 18 | ACKR3 | Atypical Chemokine Receptor 3 | 57007 | ENSG00000144476 |
| 19 | IL10 | Interleukin 10 | 3586 | ENSG00000136634 |
| 20 | CXCL10 | Interferon-Inducible Cytokine IP-10 | 3627 | ENSG00000169245 |
| 21 | C3 | Complement C3 | 718 | ENSG00000125730 |
| 22 | MKI67 | Marker of Proliferation Ki-67 | 4288 | ENSG00000148773 |
| 23 | HBEGF | Heparin Binding EGF Like Growth Factor | 1839 | ENSG00000113070 |
| 24 | C9orf47 | Sphingosine-1-Phosphate Receptor 3 | 286223 | ENSG00000186354 |
| 25 | CD40 | TNFRSF5 | 958 | ENSG00000101017 |
| 26 | EREG | Epiregulin | 2069 | ENSG00000124882 |

(continued)

|  | Gene symbol | Protein name | GeneI D | Gene ID (Ensembl) |
|---|---|---|---|---|
| 27 | CXCL9 | Monokine Induced by Gamma Interferon | 4283 | ENSG00000138755 |
| 28 | SERPINE1 | Plasminogen Activator Inhibitor Type 1 | 5054 | ENSG00000106366 |

The list of the cell surface biomarkers is given in Table 4, Table 5 and Table 6 below, respectively.

Table 4. First priority cell surface biomarkers

|  | Cluster of Differentia tion (CD) or name | UniProt Entry Name | Comments |
|---|---|---|---|
| 1 | CD11b | P11215 ITAM_HUMAN | Integrin Alpha M (ITGAM); the alpha subunit of Mac-1 (Macrophage-1 antigen), the CR3 complement receptor. Consists of CD 11b and CD18. A human cell surface receptor, found on polymorphonuclear leukocytes (mostly neutrophils), NK cells, and mononuclear phagocytes like macrophages, which is capable of recognizing and binding to many molecules found on the surfaces of invading bacteria. |
| 2 | CD62L | P14151 YAM1_HUMAN | L-selectin; a cell adhesion molecule found on leukocytes |
| 3 | CD86 | P42081 CD86_HUMAN | also referred to as B7.2, one of the B7 molecules; when bound to CD28 on T-cells, can provide the costimulatory effect. Causes up-regulation of a high affinity IL-2 receptor allowing T cells to proliferate |
| 4 | CD117 | P10721 KIT_HUMAN | C-KIT, the receptor for Stem Cell Factor, a glycoprotein that regulates cellular differentiation, particularly in hematopoiesis |
| 5 | CD144 | P33151 CADH5 _HUMAN | VECADH; a calcium-dependent adhesion molecule at intercellular junctions, found mainly in the vascular endothelium. CD144 is present on some leucocvtes as well. |
| 6 | CD177 | Q8N6Q3 CD177_HUMAN | NB1 GP; Highly expressed in normal bone marrow, also in granulocytes of patients with polycythemia vera (PV) and with essential thrombocythemia (ET) |
| 7 | CD202b | Q02763 TIE2_HUMAN | Angiopoietin-1 receptor, TEK or TIE2; TEK receptor tyrosine kinase is expressed almost exclusively in endothelial cells and some monocytes/macrophages. The ligand for the receptor is angiopoietin-1. |

Table 5. Second priority cell surface biomarkers

| | Cluster of Differentiatio n (CD) or name | Swiss Prot Access Number | Comments |
|---|---|---|---|
| 8 | FcεRI | Multiprotein complex | The high-affinity IgE receptor, also known, or Fc epsilon RI, is the high-affinity receptor for the Fc region of immunoglobulin E (IgE), an antibody isotype involved in the allergy disorder and parasites immunity. It is constitutively expressed on mast cells and basophils and is inducible in eosinophils. FcεRI is a tetrameric receptor complex consisting of one alpha (FcεRIα - antibody binding site), one beta (FcεRIβ - which amplifies the downstream signal), and two gamma chains (FcεRIγ - the site where the downstream signal initiates) connected by two disulfide bridges on mast cells and basophils. |
| 9 | HLA-DR | Multiple isoforms | HLA-DR is an MHC class II cell surface receptor encoded by the human leukocyte antigen complex on chromosome 6 region 6p21.31. The complex of HLA-DR (Human Leukocyte Antigen - DR isotype) and peptide, generally between 9 and 30 amino acids in length, constitutes a ligand for the T-cell receptor (TCR). |
| 10 | CD69 | Q07108 D69_HUMAN | An early activation marker on T cells and NK cells |
| 11 | CD101 | Q93033 GSF2_HUMAN | Also known as IGSF2 or V7. It participates in human T-cell activation and is expressed by human skin dendritic cells |
| 12 | CD163 | Q86VB7 C163A_HUMAN | M130; HbSR; RM3/1 antigen; A glycoprotein endocytic scavenger receptor for haptoglobin-hemoglobin complexes. Found specifically on monocytes/macrophages and some dendritic cells. Involved in anti-inflammatory processes. |
| 13 | CD170 | 015389 SIGL5_HUMAN | SIGLEC5 (Sialic acid-binding Ig-like lectin 5); putative adhesion molecule that mediates sialic-acid dependent binding to cells |
| 14 | CD274 | Q9NZQ7 PD1L1_HUMAN | PDL1 or PDCD1L1 (Programmed Cell Death 1 Ligand 1); expressed on T cells, NK cells, macrophages, myeloid DCs, B cells, epithelial cells, and vascular endothelial cells and others. Ligand for PD1 (CD279) and also CD80 (B7-1). Formation of PD 1/PDL1 or B7-1/PDL1 complexes transmit an inhibitory signal reducing proliferation of CD8+ T cells at lymph nodes. Anti-PDL1 drugs used ion cancer therapy |

Table 6. Third priority cell surface biomarkers

| | Cluster of Differentiation (CD) or name | Swiss Prot Access Number | Comments |
|---|---|---|---|
| 15 | CD3 | Multiprotein complex | Signaling component of the T cell receptor (TCR) complex. The complex contains a CD3γ chain, a CD3δ chain, and two CD3ε chains. |
| 16 | CD14 | P08571 CD14_HUMAN | LPSR; a membrane protein found on macrophages which binds to bacterial lipopolysaccharide |

(continued)

| | Cluster of Differentiation (CD) or name | Swiss Prot Access Number | Comments |
|---|---|---|---|
| 17 | CD15 | Not a protein | Lewis x; a carbohydrate adhesion molecule that mediates phagocytosis and chemotaxis, found on neutrophils; expressed on some B-cell chronic lymphocytic leukemias, acute lymphoblastic leukemias, and most acute nonlymphocytic leukemias. It is also called Lewis x and SSEA-1 (stage specific embryonic antigen 1) and represents a marker for murine pluripotent stem cells, in which it plays an important role in adhesion and migration of the cells in the preimplantation embryo. |
| 18 | CD16 | P08637 CG3A_HUMAN | FcγRIII; a low-affinity Fc receptor for IgG. Found on NK cells, macrophages, and neutrophils |
| 19 | CD19 | P15391 CD19_HUMAN | B4; B-lymphocyte surface antigen B4, component of the B-cell co-receptor: highly represented in B-cell malignancies, CD19 is the target of several CAR-T and mAb cancer drugs in development e.g. Juno JCAR015, Kite KTE-C19 CAR, Novartis CTL019, Morphosys MOR208, Macrogenics MGD011, Affimed AFM11 |
| 20 | CD20 | P11836 CD20_HUMAN | a type III transmembrane protein found on B cells that forms a calcium channel in the cell membrane allowing for the influx of calcium required for cell activation; expressed in B-cell lymphomas, hairy cell leukemia, and B-cell chronic lymphocytic leukemia. |
| 21 | CD56 | P13591 NCAM1_HUMAN | 140 kD isoform of NCAM (neural cell adhesion molecule), a marker for natural killer cells and some T-lymphocytes |
| 22 | CD66b | P31997 CEAM8_HUMAN | CEACAM8 (Carcinoembryonic antigen-related cell adhesion molecule 8) |

Three main clinically relevant biological subtypes (Estrogen receptor progesterone receptor positive (ER+, PR+); Human Epidermal Growth Factor Receptor 2 amplified (HER2+); triple negative breast cancer (TNBC; i.e. ER-, PR- and HER2-) and five main multiple molecular subtypes (e.g. Luminal A, Luminal B, HER2+, basal-like, normal like) that overlap largely, but not fully with the clinical subtypes have been defined. Primary breast cancer can present histologically as non-invasive ductal or lobular carcinoma (DCIS, ductal carcinoma in situ; LCIS, lobular carcinoma in situ) ; as invasive ductal or lobular carcinoma (ICS, invasive ductal carcinoma; LCS invasive lobular carcinoma); at different degrees of transformation (Grade 1, 2, 3). Brest cancer can recur locally (local relapse), or at distant sites (metastatic relapse). During progression or relapse, the biological and molecular characteristics can be retained, or the cancer may evolve toward a more aggressive and therapy resistance phenotype (e.g. loss or estrogen receptor expression or function, acquisition of additional genetic mutations, e.g. PI3KCA).

It is of interest that the method and kit of the invention detect all primary breast cancer subtypes at the earliest possible stages (non-invasive, minimally invasive, size < 2 cm). It is also of interest that the test detects all subtypes of metastatic breast cancer at the earliest possible stage (minimal burden).

Early detection of primary breast cancer is associated with decreased breast cancer-related mortality as it allows to remove the lesion as a smaller size and start an adjuvant therapy if necessary, thereby reducing the risk of local invasion and metastatic spreading.

Early detection of metastatic breast cancer allows the oncologist to adapt the adjuvant therapy, to resume or adapt a curative-intent or palliative therapy at a smaller tumor burden (compared to late detection), thereby reducing the risk of therapy resistance development and lethal organ disruption and preventing or delaying death.

Thus, it is another object of the invention to provide for a primary breast cancer detection method for a female subject, comprising:

(a) measuring in a biologic sample obtained from said female subject the expression level of transcriptomic (mRNA) markers of a first panel comprising the combination of SOX4, TNFSF10, CD3G, and NR3C2 and cell surface biomarkers specific for the immune response elicited by breast cancer at its different stages of a second panel comprising the combination of CD11b, CD62L, CD177 and CD202b;

(b) calculating a probability score based on the measurement of step (a); and

(c) ruling out breast cancer for said female subject if the score in step (b) is lower than a predetermined score; or

(d) ruling in the likelihood of breast cancer for said female subject if the score in step (b) is higher than a pre-determined score.

Preferably, the primary breast cancer detection method further comprises additional transcriptomic (mRNA) markers and cell surface biomarkers specific for the immune response elicited by breast cancer at its different stages are as identified above and reproduced in Tables 2-3 and 5-6 (second and third priority lists).

Also provided is a metastatic or recurrent breast cancer detection method for a female subject, comprising:

(a) measuring in a biologic sample obtained from said female subject the expression level of transcriptomic (mRNA) markers of a first panel comprising the combination of SOX4, TNFSF10, CD3G, and NR3C2 and cell surface biomarkers specific for the immune response elicited by breast cancer at its different stages of a second panel comprising the combination of CD11b, CD86, CD117, CD144 and CD202b;

(b) calculating a probability score based on the measurement of step (a); and

(c) ruling out breast cancer for said female subject if the score in step (b) is lower than a predetermined score; or

(d) ruling in the likelihood of breast cancer for said female subject if the score in step (b) is higher than a pre-determined score.

Preferably, the metastatic or recurrent breast cancer detection method further comprises additional transcriptomic (mRNA) markers and cell surface biomarkers specific for the immune response elicited by breast cancer at its different stages are as identified above and reproduced in Tables 2-3 and 5-6 (second and third priority lists).

Also envisioned is a primary breast cancer detection kit for use in the detection, stratification and/or monitoring of a likelihood of breast cancer in a female subject from a peripheral blood sample, said kit comprising:

- at least one probe for measuring the expression level of transcriptomic (mRNA) marker of a first panel of genes comprising the combination of SOX4, TNFSF10, CD3G, and NR3C2; and
- at least one probe and/or specific detection reagent for measuring the expression level of cell surface biomarker specific for the immune response elicited by breast cancer at its different stages of a second panel comprising the combination of CD11b, CD62L, CD177 and CD202b.

Preferably, said kit for use in the detection of primary breast cancer further comprises additional transcriptomic (mRNA) markers and cell surface biomarkers specific for the immune response elicited by breast cancer at its different stages are as identified above and reproduced in Tables 2-3 and 5-6 (second and third priority lists).

It is also the object of the present invention to provide a metastatic or recurrent breast cancer detection kit for use in the detection, stratification and/or monitoring of a likelihood of breast cancer in a female subject from a peripheral blood sample, said kit comprising:

- at least one probe for measuring the expression level of transcriptomic (mRNA) marker of a first panel of genes comprising the combination of SOX4, TNFSF10, CD3G, and NR3C2; and
- at least one probe and/or specific detection reagent for measuring the expression level of cell surface biomarker specific for the immune response elicited by breast cancer at its different stages of a second panel comprising the combination of CD11b, CD86, CD117, CD144 and CD202b.

Preferably, said kit for use in the detection of metastatic breast cancer further comprises additional transcriptomic (mRNA) markers and cell surface biomarkers specific for the immune response elicited by breast cancer at its different stages are as identified above and reproduced in Tables 2-3 and 5-6 (second and third priority lists).

The risk of developing or relapse breast cancer can be detected by examining an "effective amount" of biomarkers like proteins, peptides, nucleic acids, polymorphisms, metabolites, and other analytes in a test sample (e.g., a subject derived sample) and comparing the effective amounts to reference or index values.

An "effective amount" can be the total amount or levels of biomarkers that are detected in a sample, or it can be a "normalized" amount, e.g., the difference between biomarkers detected in a sample and background noise. Normalization methods and normalized values will differ depending on the method by which the biomarkers are detected.

Preferably, mathematical algorithms can be used to combine information from results of multiple individual biomarkers into a single measurement or index. Subjects identified as having an increased risk of breast cancer can optionally be selected to receive treatment regimens, such as the ones defined above.

Applicants preferably applies the use of a classification algorithms, and methods of risk index construction, utilizing pattern recognition features, including established techniques such as the Kth-Nearest Neighbor, Boosting, Decision Trees, Neural Networks, Bayesian Networks, Support Vector Machines, and Hidden Markov Models are encompassed by or within the ambit of the present invention, as is the use of such combination to create single numerical "risk indices" or "risk scores" encompassing information from multiple biomarkers inputs.

**Mathematical model:**

Biomarkers of interest selection:

Several methods can be combined to identify the biomarkers of interest. The statistical analyzes specific to each biomarker measurement method make it possible to measure the "differential values" of each of the biomarkers between the samples of the different groups (healthy, healthy cancer, relapse, metastatic). Statistical significance for each candidate can be determined by calculating p-value or fold-change.

Since some of the selected biomarkers might be highly correlated, a solution is to use penalized logistic regression (L1 and/or L2) to select it during the probability score calculation (see Example 4).

**Probability score:**

The probability score can be calculated according to any method known in the art. For example, the probability score is calculated from a logistic regression prediction model applied to the measurements, e.g. biomarkers normalized amounts or biomarkers indices. The probability score may be calculated by:

$$\mathrm{LOG}\left(\frac{P(y_i = 1)}{1 - P(y_i = 1)}\right) = \beta_0 + \beta_1 . x_{1_i} + \cdots + \beta_n . x_{n_i}$$

where $x_{ni},i$ is a measured value for the biomarker n and subject i and $(\beta_0, \beta_1, ..., \beta_n)$ is a vector of coefficients with $\beta_0$ a panel-specific constant, and $\beta n$ is the corresponding logistic regression coefficient of the biomarker n.

For example, the presence of a primary breast cancer can be determined as illustrated in Example 4.

Penalized logistic regression models may be validated directly on a training set or by non-overlapped bootstrap method: X random datasets were drawn with replacement from training set; each dataset had the same size as the training set. The model may be re-fit at each bootstrap and validated with the out-of-bag samples.

Fitted logistic regression models are tested by asking whether the model can correctly predict the clinical outcome using patient data other than that with which the logistic regression model was fitted, but having a known clinical outcome. After training, the model output from 0 (control) to 1 (cancer) can be calculated in blind fashion by the average error of all N predictions (a validation group).

Various statistical analysis software can be used for building logistic regression models.

**Performances (sensitivity, specificity) estimation**

The application of logistic regression to biological problems is routine in the art. Based on the output values, the receiver operating characteristic (ROC) curve can be built to calculate the outcome of clinical prediction: specificity and sensitivity of CRC cancer detection.

They are statistical measures of the performance of a binary classification test. Sensitivity measures the proportion of actual positives which are correctly identified as such (e.g., the percentage of sick people who are correctly identified as having the condition). Specificity measures the proportion of negatives which are correctly identified (e.g., the percentage of healthy people who are correctly identified as not having the condition). A perfect predictor would be described as 100% sensitive (i.e., predicting all people from the sick group as sick) and 100% specific (i.e., not predicting anyone from the healthy group as sick). However, any predictor will possess a minimum error bound.

**Definitions:**

"Penalized logistic regression":

Penalized logistic regression is based on mathematical equation derived from logistic regression. More specifically, penalized logistic regression is a ridge regression for logistic model with L2-norm or L1-norm penalty. To estimate the parameters in this method a quadratic (L2) or/and L1-norm penalty is added on the log-likelihood that should be maximized. To choose the best value of $\lambda 1$ and $\lambda 2$, the cross-validation is used with the AIC criteria. To fit the penalized logistic model, the following algorithms (packages in R Cran, statistical software) can be used: glmpath (Park M. Y and Hastie T. (2006) An L1 Regularization-path Algorithm for Generalized Linear Models. A generalization of the LARS algorithm for GLMs and the Cox proportional hazard model), penalized (Goeman, J. (2010) L1 (lasso) and L2 (ridge) penalized estimation in GLMs and in the Cox model) and glmnet (Hasti, T., Tibshirani and R., Friedman, J. (2010). Lasso and elastic-net regularized generalized linear models) with different tuning parameters.

"Sensitivity, specificity, PPV, NPV":

The term "sensitivity of a test" refers to the probability that a test result will be positive when the disease is present in the patient (true positive rate). This is derived from the number of patients with the disease who have a positive test result (true positive) divided by the total number of patients with the disease, including those with true positive results and those patients with the disease who have a negative result, i.e., false negative.

The term "specificity of a test" refers to the probability that a test result will be negative when the disease is not present in the patient (true negative rate). This is derived from the number of patients without the disease who have a negative test result (true negative) divided by all patients without the disease, including those with a true negative result and those patients without the disease who have a positive test result, e.g. false positive.

The term "positive predictive value" (PPV) refers to the probability that a positive result correctly identifies a patient who has the disease, which is the number of true positives divided by the sum of true positives and false positives.

The term "negative predictive value" or "NPV" refers to the probability that a negative test correctly identifies a patient without the disease, which is the number of true negatives divided by the sum of true negatives and false negatives. Like the PPV, it also is inherently impacted by the prevalence of the disease and pre-test probability of the population intended to be tested. A positive result from a test with a sufficient PPV can be used to rule in the disease for a patient, while a negative result from a test with a sufficient NPV can be used to rule out the disease, if the disease prevalence for the given population, of which the patient can be considered a part, is known.

"Significant":

"Statistically significant" means that the alteration is greater than what might be expected to happen by chance alone. Statistical significance can be determined by any method known in the art. For example, statistical significance can be determined by p-value. The p-value is a measure of probability that a difference between groups during an experiment happened by chance. ($P(z \geq zobserved)$). For example, a p-value of 0.01 means that there is a 1 in 100 chance the result occurred by chance. The lower the p-value, the more likely it is that the difference between groups was caused by treatment. An alteration is considered to be statistically significant if the p-value is at least 0.05. Preferably, the p-value is 0.04, 0.03, 0.02, 0.01, 0.005, 0.001 or less. As noted below, and without any limitation of the invention, achieving statistical significance generally, but not always, requires that combinations of several biomarkers be used together in panels and combined with mathematical algorithms in order to achieve a statistically significant biomarkers index.

"ROC, AUC":

A Receiver Operating Characteristics ("ROC") curve can be used as an indicator that allows representation of the sensitivity and specificity of a test, assay, or method over the entire range of test (or assay) cut points with just a single value. See, e.g., Shultz, "Clinical Interpretation Of Laboratory Procedures," chapter 14 in Teitz, Fundamentals of Clinical Chemistry, Burtis and Ashwood (eds.), 4th edition 1996, W.B. Saunders Company, pages 192-199; and Zweig et al., "ROC Curve Analysis: An ROC curve is an x-y plot of sensitivity on the y-axis, on a scale of zero to one (e.g., 100%), against a value equal to one minus specificity on the x-axis, on a scale of zero to one (e.g., 100%).

Thus, a ROC curve is a plot of the true positive rate against the false positive rate for that test, assay, or method. To construct the ROC curve for the test, assay, or method in question, subjects can be assessed using a perfectly accurate or "gold standard" method that is independent of the test, assay, or method in question to determine whether the subjects are truly positive or negative for the disease, condition, or syndrome (for example, coronary angiography is a gold standard test for the presence of coronary atherosclerosis). The subjects can also be tested using the test, assay, or method in question, and for varying cut points, the subjects are reported as being positive or negative according to the test, assay, or method. The sensitivity (true positive rate) and the value equal to one minus the specificity (which value equals the false positive rate) are determined for each cut point, and each pair of x-y values is plotted as a single point on the x-y diagram. The "curve" connecting those points is the ROC curve. The ROC curve is often used in order to determine the optimal single clinical cut-off or treatment threshold value where sensitivity and specificity are maximized. Such a situation represents the point on the ROC curve that describes the upper left corner of the single largest rectangle which can be drawn under the curve.

The total area under the curve ("AUC") is the indicator that allows representation of the sensitivity and specificity of a test, assay, or method over the entire range of cut points with just a single value. The maximum AUC is one (a perfect test) and the minimum area is one half (e.g. the area where there is no discrimination of normal versus disease). The closer the AUC is to one, the better is the accuracy of the test. It should be noted that implicit in all ROC and AUC is the definition of the disease and the post-test time horizon of interest.

As defined herein, a "high degree of diagnostic accuracy" means a test or assay wherein the AUC (area under the ROC curve for the test or assay) is at least 0.70, preferably at least 0.75, more preferably at least 0.80, preferably at least 0.85, more preferably at least 0.90, and most preferably at least 0.95.

**Normalization , Benjamini correction for multiple testing:**

Use of Benjamini-Hochberg method for calculating the false discovery rate in a clinical or diagnostic assay Am. J. Public Health 86(5): 628-629).

Normalization refers to a collection of processes that are used to adjust data means or variances for effects resulting from systematic non-biological differences between arrays, subarrays (or print-tip groups), and dye-label channels. An array is defined as the entire set of target probes on the chip or solid support. A subarray or print-tip group refers to a subset of those target probes deposited by the same print-tip, which can be identified as distinct, smaller arrays of proves within the full array. The dye-label channel refers to the fluorescence frequency of the target sample hybridized to the chip. Experiments where two differently dye-labeled samples are mixed and hybridized to the same chip are referred to in the art as "dual-dye experiments", which result in a relative, rather than absolute, expression value for each target on the array, often represented as the log of the ratio between "red" channel and "green channel." Normalization can be performed according to ratiometric or absolute value methods. Ratiometric analyses are mainly employed in dual-dye experiments where one channel or array is considered in relation to a common reference. A ratio of expression for each target probe is calculated between test and reference sample, followed by a transformation of the ratio into log 2(ratio) to symmetrically represent relative changes. Absolute value methods are used frequently in single-dye experiments or dual-dye experiments where there is no suitable reference for a channel or array. Relevant "hits" are defined as expression levels or amounts that characterize a specific experimental condition. Usually, these are nucleic acids or proteins in which the expression levels differ significantly between different experimental conditions, usually by comparison of the expression levels of a nucleic acid or protein in the different conditions and analyzing the relative expression ("fold change") of the nucleic acid or protein and the ratio of its expression level in one set of samples to its expression in another set.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this specification, each of which is incorporated herein by reference in its entirety.

The foregoing description will be more fully understood with reference to the following Examples. Such Examples, are, however, exemplary of methods of practicing the present invention and are not intended to limit the scope of the invention.

**Examples**

**Example 1:**

**Bevacizumab specifically decreases elevated levels of circulating KIT+CD11b+ cells and IL-10 in metastatic breast cancer patients**

Applicants performed a case-control clinical study to demonstrate that metastatic BC (mBC) patients have elevated levels of circulating TIE2$^+$CD11b$^+$ and KIT$^+$CD11b$^+$ cells (Figure 1) and IL-10 and that the anti-Vascular Endothelial Growth Factor (VEGF) antibody bevacizumab (Avastin) specifically reverses them to values observed in clinically tumor free patients [9]. Applicants also analyzed the transcriptome of CD11b$^+$ myeloid cells derived from four mBC patients and four healthy donors (HD). Applicants using a genome wide screen identified 271 genes significantly differentially expressed between mBC patients and HD. In particular they observed that CD11b$^+$ cells from mBC patients expressed genes of M2 monocytes' activation state (i.e. CD163, ARG1, and IL10) (**Figure 2**). This important demonstrated the presence of circulating KIT$^+$CD11b$^+$ in patients with metastatic breast cancer, and that that in these patients CD11b$^+$ cells have a different transcriptional profile compared to CD11b$^+$ cells in healthy individuals. Further it revealed the phenotypical and functional plasticity of CD11b[+] in response to anti-angiogenic therapy (anti-VEGF).

**Example 2:**

**Circulating immune cell populations related to primary breast cancer, surgical removal and radiotherapy revealed by flow cytometry analysis**

**Summary**

Advanced breast cancer (BC) impact immune cells in the blood but whether such effects may reflect the presence of early BC and its therapeutic management remains elusive. To address this question, applicants used multiparametric flow cytometry to analyze circulating leukocytes in patients with early BC (n=13) at time of diagnosis, after surgery and after adjuvant radiotherapy, compared to healthy individuals. Data were analyzed using a minimally supervised approach based on FlowSOM algorithm and validated manually. At time of diagnosis, BC patients have an increased frequency of CD117[+] Granulocytic-Myeloid Derived Suppressor Cells (G-MDSC), which was significantly reduced after tumor removal. Adjuvant radiotherapy increased the frequency of CD45RO+ memory CD4[+] T cells and CD4+ regulatory T cells. FlowSOM algorithm analysis revealed several unanticipated populations, including cells negative for all markers tested, CD11b[+]CD15low, CD3[+]CD4[-]CD8[-], CD3[+]CD4[+]CD8[+], CD3[+]CD8[+]CD127[+]CD45RO[+] cells, associated with BC or radiotherapy. This study revealed changes in blood leukocytes associated with primary BC, surgical removal and adjuvant radiotherapy. Specifically, it identified increased levels of CD117[+] G-MDSC, memory and regulatory CD4[+] T cells as potential biomarkers of BC and radiotherapy, respectively. Importantly, the study demonstrates the value of unsupervised analysis of complex flow cytometry data to unravel new cell populations of potential clinical relevance.

**Introduction**

Breast cancer (BC) is the most frequent cancer and main cause of cancer-related mortality for women in industrialized countries. Three clinically relevant biological BC subtypes (i.e. Estrogens/Progesterone Receptor (ER/PR) positive, Human Epidermal growth factor Receptor 2 (HER2) amplified and triple negative), and multiple molecular subtypes (e.g. Luminal A/B, HER2, basal like, normal like) with distinct features and clinical outcomes, have been defined and characterized.

Early detection and surgery in combination with adjuvant treatments tailored on biological and molecular subtypes, have improved patients' survival by about 30% in the past three decades. Goal of adjuvant therapy, including radiotherapy, is the eradication of tumor cells that disseminated before diagnosis and surgery. Some of these disseminated tumor cells (DTC), however, will escape therapy and later progress to form metastases, which in most patients represents the main causes of cancer-related death. After breast-conserving surgery, radiotherapy reduces the risk of BC recurrence and death. Among women with operable BC, randomized trials have demonstrated equivalent disease-free and overall survival between mastectomy and breast-conserving surgery followed by radiotherapy alone and/or hormonal, anti-HER2, or chemotherapy.

Mammography is the standard approach for the detection of asymptomatic BC. In spite of its benefits in reducing BC specific mortality, mammography has some important limitations: low specificity and sensitivity; risk of over-diagnosis; risk of inducing BC due to X-ray exposure, particularly in patients with defective DNA repair genes; not recommended before the age of 50 in spite of the fact that 20-25% of all BCs appear before this age. There is therefore an unmet need for complementary or alternative methods for the detection of asymptomatic, early BC. Circulating tumor cells (CTC), cell free tumor-derived DNA, mRNA and miRNA, proteins, autoantibodies and metabolites are being explored as candidate blood-based biomarkers for BC detection, diagnosis or monitoring, but so far none entered routine clinical practice. Similarly, there are no effective blood-based biomarkers to actively assess patients' response to treatment and monitoring disease state after therapy. Also, the most used in clinical practice biomarker protein such as CA 15-3 is not specific and sensitive in early breast cancer diagnosis. Tumors, including BC, mobilize and recruit immuno-inflammatory cells to their microenvironment. Monocytic and granulocytic cells, mostly immature forms thereof, as well as lymphocytes, contribute to cancer progression by promoting immunosuppression, angiogenesis, cancer cell survival, growth, invasion and metastasis. Applicants have previously shown that metastatic BC patients have elevated frequencies of TIE2[+]CD11b[+] and CD117[+]CD11b[+] leukocytes circulating in the blood, and that circulating CD11b[+] cells express higher mRNA levels of the M2 polarization markers CD163, ARG1 and IL-10. Treatment with paclitaxel in combination with bevacizumab decreased the frequency CD117[+]CD11b[+] leukocytes, IL-10 mRNA levels in CD11b[+] cells and IL-10 protein in plasma. Applicants therefore considered that blood circulating leukocytes, or sub-population thereof, may reflect cancer-relevant immuno-inflammatory events that may be further explored as BC-associated biomarkers.

**Results:**

**Increased frequency of CD117$^+$ granulocytic-MDSCs in the peripheral blood of patients with newly diagnosed non-metastatic BC:**

Based on observations made in metastatic BC patients, Applicants hypothesized that an increased frequency of circulating CD11b$^+$ cells expressing CD117 and/or displaying a M2 activation phenotype, may also occur in patients with early BC. To test this hypothesis, applicants monitored the frequency of MDSC cells in the blood of non-metastatic BC patients (cT1-4, N0-1, M0) at time of diagnosis using Flow Cytometry. Aged-matched women without BC served as control population (healthy donors - HDs). Monocytes MDSC (Mo-MDSC) were defined as CD11b$^+$CD33$^+$CD14$^{high}$CD15$^-$ and granulocytic MDSC (G-MDSC) as CD11b$^+$CD33$^+$CD14$^{low}$CD15$^+$ cells. In both cell populations applicants monitored the expression of CD117, the receptor for Kit-ligand/stem cell factor widely present in hematopoietic progenitors' cells, and CD163, a M2 polarization marker in monocytes. In order to avoid investigator-associated biases and variability in the results inherent to supervised manual analysis of flow cytometry data, Applicants developed a minimally supervised, standardized analytical workflow based on the FlowSOM algorithm, in complement to conventional manual gating and supervised analysis **(Figure 3)**.

Cells clusters revealed by standardized analytical workflow, were considered of interest when their frequency was more than 10% different between HDs and cancer patients. Some of the 14 analyzed clusters corresponded to non-standard populations, for example those negative for all tested markers or expressing unanticipated marker combinations **(Figure 4)**. These populations would have been missed by conventional supervised gating and analysis driven by the marker combination of interest. Interestingly, Applicants observed a significant increase in the frequency of CD117$^+$ cells among the circulating G-MDSC population in cancer patients relative to HDs. Applicants observed a similar (but non-significant) trend in the frequency of Mo-MDSC cells, albeit at lower frequency. In addition, the frequencies of some non-classical cell populations, such as those expressing none of the markers of interest (Cluster 13 and 3) or a CD11b$^+$CD15$^{low}$ cell population (Cluster 22+9), were significantly different between BC patients and HDs. No significant changes were observed for CD163$^+$ cells in both G-MDSC and Mo-MDSC populations **(Figure 5)**.

**Non-standard CD3 expressing cells are present with increased frequency in the peripheral blood of newly diagnosed BC patients**

In parallel the applicants monitored the presence of selected lymphocyte populations in both groups. By conventional supervised analysis the applicants observed no differences in classical CD3$^+$CD4$^+$ T cells, CD3$^+$CD8$^+$ T cells and CD3$^+$CD4$^+$CD25$^+$CD127$^-$ regulatory T cells (Tregs). Likewise, Applicants observed no changes in the frequency of memory (CD45RO$^+$CD45RA$^-$) or naive (CD45RA$^+$CD45RO$^-$) T cells within the same lymphocyte populations.

In contrast, FlowSOM analysis performed on lymphocytes revealed 21 populations that were more then 10% differentially represented between HDs and cancer patients. A population of cells of the size of lymphocytes, but negative for CD3, CD4 or CD8 expression (Cluster 24 + 29) was significantly less represented in cancer patients relative to HDs. CD3$^+$CD8$^+$ T cells expressing CD127 and CD45RO markers (Cluster 3+7) are present at significantly higher frequency in cancer patients. A cell cluster expressing CD3, but not CD4 or CD8 (Cluster 20) was fond more represented in cancer patients relative to HDs. Importantly, all these populations would have been missed by supervised analysis based on standard marker combinations defining classical lymphocyte subpopulations.

Taken together these results reveal an increased frequency of peripheral blood CD117$^+$ G-MDSC in non-metastatic BC patients at time of diagnosis, as well as significant changes in the frequency of myeloid and lymphocytic cell populations expressing unconventional marker combinations. They also demonstrate that FlowSOM-based analysis can identify cell populations that would have been likely missed by supervised analysis.

No detectable changes in the expression level of transcripts for M2 polarization markers Applicants previously reported that transcripts of M2-associated genes were expressed at higher levels circulating CD11b$^+$ cells in metastatic BC patients compared to HDs. Applicants therefore analyzed expression of mRNA for CD117 and the M2 markers IL-10, fibronectin-1 (FN1) and arginase 1 (ARG1) in total leukocytes from cancer patient and HDs. No differences in expression levels were observed.

**A proof of concept study to monitor the effects of surgical tumor removal and adjuvant radiotherapy on circulating immune cells in BC patients**

The differences observed in myelomonocytic and lymphocytic populations in BC patients at time of diagnosis relative to HDs, raised the question whether tumor removal and/or adjuvant therapy may reverse these changes, or induce additional ones. To address this question, Applicants performed a proof of concept study, by taking advantage of the fact that the investigated patients were scheduled for breast conservative tumor removal and adjuvant radiotherapy as part of their standard treatment. Adjuvant radiotherapy was selected as therapy of choice as systemic effects on the immune system have been reported, while on the other side chemotherapy was excluded in order to avoid that myelosuppressive effects induced by chemotherapy could non-specifically impact the results. To search for potential changes in cell populations in response to surgery and radiotherapy applicants analyzed G-MDSC and Mo-MDSCs as well as lymphocytes at three time points : after surgery/before radiotherapy start (1 _PostOP), at the end of radiotherapy (6 weeks; 2_Post_RTX_6w) and at 6-8 weeks after the end of radiotherapy (12-14 weeks; 3_Post_RTX_12w). Results were compared to values obtained at time of diagnosis (0_PreOP).

Tumor removal increased the frequency Mo-MDSCs and G-MDSCs but decreased CD117[+] G-MDSCs and radiotherapy induced changes in non-standard myeloid cell populations **(Figure 6).** Using FlowSOM workflow of analysis Applicants observed distinct expression profiles at the four time-points globally visualized by -Distributed Stochastic Neighbor Embedding (tSNE). Seventeen cell clusters were found highly differentially represented in one group compared to the other groups. Surprisingly, when looking at the expression profiles of each cluster of interest, the majority of them was lacking CD33 expression, suggesting that this marker may not be suitable to analyze the monocytes fraction.

After tumor removal and at the end of radiotherapy the frequency of both Mo-MDSCs and G-MDSCs was significantly increased relative to values at time of diagnosis, and returned to pre-therapy levels 6-8 weeks after the end of radiotherapy. Strikingly, within the G-MDSCs population, the fractions of CD117 expressing cells significantly decreased after tumor removal and this decrease persisted after the end of radiotherapy. Surgery had no impact on the fraction of CD163[+] G-MDSCs population. Radiotherapy itself had an impact on G-MDSC expressing CD163, but not CD117 **(Figure 7).**

The presence of one particular cell cluster expressing only CD11b and CD15 (Pop 1, 6 and 7) clearly decreased after radiotherapy. Another population expressing CD11b but lacking expression of all tested markers significantly increased during and after radiotherapy (Pop 16, 18, 12, 15 and 26). The latter observation, suggests that some populations defined by non-standard marker combinations may be potentially interesting candidates to investigate further with an extended panel or markers.

Analysis of total blood leukocytes for CD117, IL-10, FN1, and ARG1 mRNA expression by RT-qPCR revealed no observable differences in their expression levels.

**Adjuvant radiotherapy increases the frequency of CD4[+] memory and regulatory T cells, and induces changes in non-standard lymphocytic populations**

Likewise, Applicants performed unsupervised analysis of the lymphocyte populations at the three time-points after surgery and radiotherapy. Visualization by tSNE revealed distinctive changes in marker expression profiles. Eleven cells clusters were found highly differentially represented in one group compared to the other ones. After tumor removal the applicants observed highly variable effects on the frequency of T lymphocyte subpopulations, most of which were inconsistent and statistically non-significant.

Among the stably differentially represented clusters at the various time-points, a CD3[+] cell population positive for CD4 and CD8 (Cluster 25), and a CD3[+]CD4[+]CD127[+]CD45RO[+] population (Cluster 41) appeared at higher frequency after treatment. Strikingly, the frequency of this CD45RO[+]RA- memory subset within the CD3[+]CD4[+] lymphocyte population was significantly and consistently increased at the end of radiotherapy and this increase was still evident 6-8 weeks later. A similar increase was also present among CD4+ regulatory T cells, corresponding to cluster 23 and 30, which also persisted after the end of radiotherapy.

**Discussion**

Mammography-based screening significantly reduces BC-related mortality, but intrinsic and practical limitations call for novel, complementary or alternatively screening approaches Blood based biomarkers exploiting cancer-derived circulating cells (CTC), DNA (ctDNA) or RNA (miRNA, mRNA) are being explored, but so far none reached clinical routine practice. Similarly, there are no valid biomarkers for monitoring patients' response to treatment or detecting relapses before they become symptomatic.

Flow cytometry is an attractive technique allowing fast, robust and cost-effective simultaneous detection of multiple parameters at single cell resolution. Applicants previously applied flow cytometry to characterize peripheral blood leukocytes in mBC patients, relative to HDs and to monitor effects of chemo- and anti-angiogenic therapies. Supervised analysis of flow cytometry data, however, has several limitations: it can only identify known cell populations based on a few markers and their combination, is not suited to identify unanticipated populations by testing all possible marker combinations, is inefficient when analyzing large and complex data sets, and is subjected to variability within and between studies, especially if studies are spread of long periods of time. To overcome these limitations, applicants implemented a new data analysis workflow using the FlowSOM algorithm allowing for a more standardized and a minimally supervised protocol. This new-way of proceeding to analyze the data generates more reproducible and less biased results over the time course of the study and prevents "investigator-specific" biases. In addition, this approach significantly increases robustness and accuracy, both crucial parameters when performing clinical studies with large datasets aimed at revealing recurrent, significant differences between several groups of patients or conditions. Importantly, it allows to identify specific differences across groups beyond pre-defined populations, which is a significant advantage in biomarker discovery efforts.

In this study applicants pursued the use of flow cytometry to analyze the phenotype and frequency of blood leukocytes in patients with non-metastatic BC at time of diagnosis, after surgical tumor removal and after adjuvant radiotherapy. Using a combination of minimally supervised (FlowSOM algorithm) and supervised (manual) analytical approaches, applicants report that: *i*) at diagnosis, BC patients have an increased frequency of circulating CD117+ Granulocytic-MDSC relative to age-matched healthy donors; *ii*) surgical tumor removal causes transient increase of G-MDSCs and M-MDSCs, and a long-lasting decrease of CD117+ G-MDSC; *iii*) radiotherapy significantly increases CD45R0+ memory T cells and CD4+ Treg cells; iv) with the FlowSOM algorithm the applicants identified additional unanticipated, non-classical cell populations differentially represented between HD and BC patients and in BC patients in response to therapy, including CD11b+CD15low, CD3+CD4-CD8-, CD3+CD4+CD8+ and CD3+CD8+CD127+CD45RO+ populations and cells negative for all markers.

CD117, the receptor for Kit-ligand/stem cell factor, is widely expressed in hematopoietic progenitor cells in the bone marrow, while virtually no CD117+ leukocytes are present in the circulation under homeostatic conditions CD117 expressing cells nevertheless appear in the circulation under pathological conditions, including cancer, acute coronary syndrome or leukemia's. The applicants have previously reported a role of CD117+ leukocytes in metastasis in the murine 4T1 metastatic BC model and the presence of CD117+CD11b+ cells in the blood of mBC patients. Here, applicants observed an increased frequency of CD117+ cells among total CD11b+ G-MDSCs in the peripheral blood of non-metastatic BC patients at time of diagnosis, compared to HD. Interestingly, the frequency of CD117+ G-MDSCs significantly dropped upon tumor removal and remained below pre-treatments levels after radiotherapy. Thus, the increased frequency of CD117+ G-MDSCs may reflect the presence of the primary tumor. No changes were observed in CD117 mRNA expression in total leukocytes. This could be due to that fact that CD117+ cells are lost during leukocyte isolation for RNA extraction (flow cytometry was performed in non-separated total whole blood), or that CD117 mRNA expression has ceased upon cell mobilization (while CD117 protein persisted at the cell surface). The latter possibility is consistent with our previous observation that mobilized CD117+ cells adoptively transferred to a recipient mouse, rapidly became CD117 negative. In contrast, the frequency of CD163+ G-MDSCs remained rather constant, with only a transient decrease during radiotherapy. The implication of this decrease is unclear as CD163 expression did not significantly differ between HD and BC patients at time of diagnosis. Nevertheless, elevated levels of CD163+ M2-polarized monocytes was reported in patients with acute pancreatitis or membranous nephropathy. After surgery and radiotherapy also no change in the mRNA expression of CD117 and ARG1, FN1, IL-10 (i.e. M2 polarization markers) was observed, owing probably to the lack of enrichment of CD11b+ cells for PCR analysis.

In cancer patients at time of diagnosis applicants observed a higher frequency of atypical T lymphocytes (CD3+CD4-CD8-) and of a population of the size of lymphocytes lacking expression of all the tested markers. These observations show that some interesting changes occur in atypical T cells or in non-T cell populations such as B cells or NK cells. Strikingly, after radiotherapy, applicants observed a steady and significant increase of the fraction of CD45RO+ memory T cells within total CD4+ T cells and within CD4+ Treg. Applicants also observed the increased presence of a T cell population expressing both CD4 and CD8 markers. This suggests that radiotherapy causes T cell activation leading to the subsequent generation of memory T cell subsets. Indeed, there is evidence that radiotherapy exerts its therapeutic effects, not only in the local treatment field, but also outside the irradiated field and at distant sites (i.e. the so called abscopal effect), at least in part, by eliciting a T cell immune response. The recent observation that combination of radiotherapy with immune checkpoint inhibitors in experimental models and cancer patients results in potent synergistic therapeutic effects further supports the involvement of T cell-dependent events and the therapeutic effects of radiotherapy. Through experimental work and mathematical modelling, it has been proposed that anti-tumor T cells can by mobilized by radiotherapy toward peripheral tissues to eliminate DTC. However, to date there is paucity of human data demonstrating specific changes in circulating T lymphocytes to support such a model. Radiotherapy was reported to cause a global reduction in circulating lymphocyte subsets in patients treated for stage I-II prostate cancer, or to induce an increase in CD4+ Treg in the peripheral blood of patients with diverse solid cancers. Low-dose radon therapy for chronic inflammatory diseases was shown to induce a long-lasting increase in circulating T cells paralleled with a reduced expression of activation markers. Thus, the observed effect of adjuvant radiotherapy on memory CD4+ T cells is novel and should be further explored in conjunction with patients' outcome, as a possible biomarkers of therapy response or efficacy.

**Conclusion**

Taken together, this human exploratory study in early, non-metastatic BC revealed changes in blood leukocyte populations associated with the presence of BC, surgical removal and adjuvant radiotherapy. Specifically, Applicants identified CD117+ G-MDSC and CD45RO+ CD4+ memory T cells correlating with the presence of the primary tumor and radiotherapy, respectively. Importantly, the study demonstrates that a minimally supervised, algorithm-based analysis of flow cytometry data is a powerful tool to reproducibly detect phenotypical changes in peripheral blood leukocytes in cancer patients. The approach also identifies non-anticipated population correlated with disease state of therapy. These results instigate the further investigation of peripheral blood leukocytes as source of reliable candidate biomarkers to detect BC, to monitor response to treatment and possibly disease progression.

### Materials and Methods

### Patients and clinical study

The study was approved by the Cantonal ethic commission for human research on Humans of Canton Ticino (CE 2967) and extended to Vaud-Fribourg-Neuchâtel, Switzerland. Applicants studied 13 female patients who were diagnosed with primary, non-metastatic BC (stage T1-4, N0-N1, M0,). All patients underwent conservative surgery and received standard fractionated adjuvant radiotherapy (2 Gy per session, total dose: 50+10 Gy). From these patients, blood samples were collected after confirmed diagnosis/before surgery, after surgery/before radiotherapy, at the end of radiotherapy (6 weeks), and 6-8 weeks after the end of the radiotherapy (week 12-15). All Patients and HDs gave written informed consent before study entry. Patients were recruited before surgery at Clinica Luganese Moncucco, Lugano, and at Hôpital Neuchâtelois, La Chaux-de-Fonds, once diagnosis was histologically confirmed. Mean age for cancer patients was 60.6 years (all patients were between 43 and 73 years old). HDs were recruited along the study, based on the following criteria: age-matched relative to BC patients, no regular medications in the last 6 months, no previous cancer diagnosis, no chronic diseases and normal blood analyses at time of recruitment.

### Blood processing

Twenty ml of peripheral venous blood was collected using Becton Dickinson (BD) Vacutainer® Blood Collection EDTA Tubes (Becton Dickinson, Franklin Lakes, NJ, USA) following manufacturer's instructions and immediately shipped by courier at room temperature to the laboratory. All analyses were performed within 24 hours after blood collection. Antibody staining was performed in whole blood (see below). Plasma and total leukocytes were isolated from the remaining blood using BD Vacutainer® CPT™ Cell Preparation Tube (Becton Dickinson) with Sodium Heparin following manufacturer's instructions of use. Plasma fraction was frozen at -80°C and isolated leukocytes were lysed in RA1 lysis buffer (Macherey-Nagel, Düren, Germany) and stored at -80°C.

### Flow cytometry

Whole blood staining's were performed within 24 hours after blood collection. Leukocytes were counted using Cell-Dyn Sapphire Hematology System (Abbott Diagnostics, Chicago, IL, USA). For staining, 1 million cells per tube were used. Directly labelled antibodies were added to whole blood and incubated for 20 minutes at 4°C, followed by 10 minutes red-blood-cells lysis (Bühlmann Laboratories, Schönenbuch, Switzerland) and washing using cold PBS. All anti-human antibodies were used at the concentrations recommended by the manufacturer: anti-CD15-PeCy7 (clone HI98), anti-CD14-Pe (clone MφP9), anti-CD163-FITC (clone GHI/61), anti-CD11b-BV510 (clone ICRF44), anti-CD33-V450 (clone WM53), anti-CD64-APCH7 (clone 10.1), anti-CD117-APC (clone YB5.B8), anti-CD45RA-PeCy7 (clone HI100), anti-CD25-Pe (clone M-A251), anti-CD4-FITC (clone RPA-T4), anti-CD8-V500 (clone SK1), anti-CD45RO-BV421 (clone UCHL1), anti-CD3-APCH7 (clone SK7), and CD127-Alexa Fluor 647 (clone HIL-7R-M21) and 7AAD (all from Becton Dickinson). BD FACSCanto II (Becton Dickinson) instrument was used to analyze samples and FlowJo 10.6.2 (Treestar Inc., Ashland, OR, USA) software and several software plugins (FlowCLEAN, downsample_V3, FlowSOM, tSNE) were used to analyze all data.

### Reverse transcription real-time PCR (RT-qPCR)

Total mRNA from total white blood cells was extracted using the NucleoSpin RNA kit from Macherey-Nagel following manufacturer's instructions (Düren, Germany). The purity and quantity of all RNA samples were examined by NanoDrop (Witec AG, Luzern, Switzerland). Total RNA was retro-transcribed using M-MLV reverse transcriptase kit following manufacturer's instructions (ThermoFisher Scientific, Waltham, Massachusetts, USA) using 500 ng of total RNA. cDNA was subjected to amplification by real time qPCR with the StepOne SYBR System (Life Technologies) using primer pairs (Eurofins Genomics, Huntsville, AL, USA) specific for the following transcripts GAPDH (GeneID: 2597);

ARG1 (GeneID: 383); IL-10 (GeneID: 3586); KIT (GeneID: 3815); FN1 (GeneID: 2335). Real-time PCR data were then analyzed using the comparative Ct method.

**Statistical analysis**

Acquired data were analyzed and graphics were generated using Prism Software (GraphPad, La Jolla, CA, USA). Statistical comparisons between cancer patients and healthy donors were performed by T-test assuming non-homogenous variance. Normality distribution of the samples was checked in case of significance and if non-Gaussian a Mann-Whitney replaced the T-test results. Statistical comparisons of all time points to observe the effect of radiotherapy were performed by one-way Analysis Of Variance (ANOVA) assuming non-homogenous variance using Tukey correction. Normality distribution of the samples was checked in case of significance and if non-Gaussian a Kruskal-Wallis assay replaced the ANOVA results. Highest and lowest values from each group were excluded. Results were considered to be significantly from $p < 0.05$. In the figures the various p values thresholds are presented as follow: $\leq 0.05 = *$, $\leq 0.01 = **$, $\leq 0.001 = ***$, $\leq 0.0001 = ****$.

**Workflow for flow cytometry unsupervised analysis**

Manual analysis of complex flow cytometry data sets is time-consuming and the results can be affected by the experience of the person performing the analysis and the expected results. In order to standardize and optimize flow cytometry data analysis process, and avoid investigator-associated biases the applicants established a new analytical workflow.

The initial preparation of clean data files to feed the algorithm was still performed partially manually. These manual steps consisted in the automatic compensation of each file, the removal of debris and dead cells based on viability dye, and the exclusion of doublets based on forward-scatter area versus high. The file is then cleaned from any event recorded under conditions of unstable flow using the FlowCLEAN algorithm of FlowJO software. Based on forward and side scattering data, all cell populations or populations of interest, as in our case, are selected for further analysis. Following this initial data cleaning, samples of interest are down-sampled using the plugin in FlowJO to normalize the number of cells between analyzed samples. Then all data are concatenated in one single file in order to analyze and compare all patients together. Finally, the FlowSOM algorithm is applied to the concatenated file for unsupervised detection of clusters representing distinct cell populations. Each cluster identified by the FlowSOM algorithm is then compared for its presence or absence in cancer patient and healthy donors, and differentially present cell clusters are investigated and manually to validate the specific expression profile and distribution.

This new analysis workflow consents a minimally supervised, robust and faster approach to analyze large and complex sets of flow cytometry data. This workflow can be applied to high number of patients avoiding time consuming manual gating and is more reproducible then the classical fully manual gating and analysis.

**Example 3:**

**Phenotypical and transcriptomics changes in immune cells circulating in the blood of patients with primary or metastatic breast cancer.**

**Introduction**

In spite of a decrease in mortality by approximately 30% over the past 30 years, breast cancer (BC) remains the leading cause of cancer-related mortality for women in industrialized countries. About one third of patients still die of the disease, due to the formation of metastases. In order to decrease breast cancer mortality, it is crucial to diagnose BC as early as possible, particular in younger women, and to prevent or treat metastases effectively.

Applicants have performed preclinical and clinical studies showing that the transcriptome and the phenotype of blood leukocytes is modulated by the presence of a primary or metastatic BC. In particular, applicants observed the appearance of cKit (CD117) and Tie2 (CD202b) - expressing CD11b$^+$ myelomonocytic, cells. In cancer patients the leukocyte transcriptome is skewed toward the expression of genes promotion cell proliferation and immunosuppression. In this study, applicants aim at identifying biomarkers associated with the presence of a primary breast cancer or relapse after initial, by characterizing the cell surface phenotype of blood leukocytes using single multiparametric cell surface phenotyping.

Applicants have performed a prospective case-controlled study in which they analyzed the phenotype and transcriptome of circulating immune cells (leukocytes) in the blood of primary or metastatic breast cancer (BC) patients and healthy women. Sixty-six patients and healthy women were enrolled in four groups: first BC diagnosis; BC patients relapse after therapy; healthy women; BC patients without relapse. All three biological BC subtypes (ER$^+$; HER2+, triple negative BC) have been investigated. The is a multicentric study in the Lemanic area and coordinated at CHUV. Twenty ml blood has been collected for analysis together with clinicopathological information.

**Results**

**Phenotypical analysis**

Forty-eight subjects were recruited and analyzed (22 primary BC, 11 metastatic BC and 15 heathy controls). Peripheral blood leukocytes were stained with 19 fluorescent labelled antibodies (CD3, CD11b, CD14, CD15, CD16, CD19, CD20, CD56, CD62L, CD66b, CD69, CD86, CD101, CD117, CD163, CD177, CD202b, CD274, HLA-DR). Labelled cells were processed by flow cytometry and subjected to supervised analysis for CD11b" myelomonocytic cell subpopulations. Applicants observed changes in the frequencies of various myelomonocytic cell populations between primary BC patients (CD177+ non-classical monocytes; CD202b+ intermediate monocytes; CD202b+ classical monocytes; CD62L+ neutrophils), metastatic BC patients (CD202b+ intermediate monocytes; CD202b+ classical monocytes; CD86+ polarized non-classical monocytes; CD117+ classical monocytes; CD144+ intermediate monocytes) and healthy donors. This study confirmed phenotypical changes in circulating leukocytes significantly associated with the presence of a primary BC or a metastatic BC (Figure 8 and Table 7).

Table 7

| CD11b+ Populations | Marker | HD vs. P | HD vs. M | P vs. M |
|---|---|---|---|---|
| % of Non-classical MC | CD177 | 0.0072 | | |
| % of Neutrophils | CD62L | 0.0001 | | |
| % of Intermediate MC | CD202b | 0.0184 | 0.0054 | |
| % of Classical MC | CD202b | 0.0055 | 0.0011 | |
| % of Intermediate MC | CD144 | | 0.0421 | |
| % Non-classical MC polarisation | CD86 | | 0.001 | 0.0029 |
| % of Classical MC | CD117 | | 0.0037 | 0.0324 |

Table 7 summarizes the comparative phenotypical changes of monocytic cells in the blood healthy donors (HD) vs primary BC patients (P); healthy donors (HD) vs metastatic BC patients (M); primary BC patients (P) vs metastatic BC patients (M). "Marker" indicates the difference-determining marker. Numbers indicate p values of Bonferroni multiple comparative tests.

Transcriptomic analyses were performed by scRNASeq on the total peripheral blood mononuclear fraction. It revealed significant differences in gene expression between healthy donors and primary BC patients. Some of the differentially expressed genes were expressed in metastatic BC patients, while some are specific to primary breast cancer patients (See example 1 and Table 9).

Table 8

| Marker | Lymphocytes | NK cells | Myelomonocytes |
|---|---|---|---|
| CD3G | <0.005-0.00001 | | |
| TNFSF10 | <0.009-0.0003 | | |
| NR3C2 | <0.04 | | <0.05 |
| SOX4 | | <0.01 | <0.01 |
| KLF12 | | <0.03 | |
| GZMH | <0.03 | | |
| LY9 | <0.01 | | |
| HLA-DOA | <0.03 | | |
| CX3CR1 | <0.01 | | |
| S1PR1 | <0.01 | | |
| HLA-DOA | <0.03 | | |

Table 8 summarizes the comparative transcriptomic changes of lymphocytes, natural killer cells and myelomonocyte cells in the blood of healthy donors vs primary BC patients. Numbers indicate p values after correction.

## Conclusion

This study confirmed that phenotypical and transcriptomic changes in circulating leukocytes are significantly associated with the presence of a primary BC or a metastatic BC. These changes in the frequency of circulating leukocytes subpopulations can be used to devise combining strategies to identify patients with primary BC relative to healthy women, and patients with metastatic BC relative to patients with primary BC or healthy women. For example, primary BC patients can be identified through altered cell surface levels of CD62L on neutrophil polymorphonuclear (PMN) granulocytes, altered cell surface levels of CD202b on intermediate and classical monocytes (icMo) and altered cell surface levels of CD177 on non-classical monocytes (ncMo). Metastatic BC patients can be identified through altered cell surface levels of CD202b on intermediate and classical monocytes (icMo), altered cell surface levels of CD117 on classical monocytes (cMo), altered cell surface levels of CD144 on intermediated monocytes (iMo) and altered cell surface levels of CD86 on non-classical monocytes (ncMo) (see Figure 9). A similar strategy can be devised based on differentially expressed genes.

## Materials and Methods

### Patients and clinical study

This was a national multicentric controlled study for observational translational research involving prospective collection of blood samples and health-related personal data from patients and healthy individuals to identify predictive biomarkers for the detection of breast cancer at any stages of the disease. The study was approved by the ethical committee for research on humans of the Cantons of Vaud, Fribourg, Valais and Neuchatel (CER-VD) under number 2020-02414. Patients and healthy individuals seen at the participating centers and whom comply with the eligibility criteria of one of the study groups have been offered to participate in this study and asked to donate blood samples according to the project schedule. To be eligible, patients consented to the collection of both biological material and clinical data. HDs were recruited along the study, based on the following criteria: age-matched relative to BC patients, no regular medications in the last 6 months, no previous cancer diagnosis, no chronic diseases and normal blood analyses at time of recruitment.

### Blood processing

Twenty ml of peripheral venous blood was collected using Becton Dickinson (BD) Vacutainer® Blood Collection EDTA Tubes (Becton Dickinson, Franklin Lakes, NJ, USA) following manufacturer's instructions and immediately shipped by courier at room temperature to the laboratory. All analyses were performed within 24 hours after blood collection. Antibody staining was performed in whole blood (see below). Flow cytometry analyses are performed on whole blood after red blood cell lysis. For sequencing, peripheral blood mononuclear cells were isolated from whole blood by magnetic activated cell sorting (MACS).

### Flow cytometry

Whole blood staining's were performed within 24 hours after blood collection. Leukocytes were counted using Cell-Dyn Sapphire Hematology System (Abbott Diagnostics, Chicago, IL, USA). For staining, 1 million cells per tube were used. Directly labelled antibodies were added to whole blood and incubated for 20 minutes at 4°C, followed by 10 minutes red-blood-cells lysis (Bühlmann Laboratories, Schönenbuch, Switzerland) and washing using cold PBS. All anti-human antibodies were used at the concentrations recommended by the manufacturer: Anti-CD101-Brilliant Violet 711 ; Anti-CD15-V450 ; Anti-CD16-PE-Cy7 ; Anti-Anti-CD274-Brilliant Violet 421 ; Anti-CD62L-BB700 ; Anti-CD86-Brilliant Violet 711; Anti-CD117-Brilliant Violet 711 ; Anti-CD11b- Anti-Alexa Fluor 488 ; Anti-CD14-Brilliant Violet 605 ; Anti-CD163-Fire 750 ; Anti-CD177-PE ; Anti-CD202b (Tie2)-PE ; Anti-CD66b Alexa Fluor 700 ; Anti-CD69-APC-Fire 750 ; Anti-HLA-DR-PE ; Anti-CD3-APC; Anti-CD19-APC; Anti-CD20-APC; Anti-CD56-APC. BD Fortessa (Becton Dickinson) instrument was used to acquire samples and FlowJo 10.6.2 (Treestar Inc., Ashland, OR, USA) software was used to analyze data.

Populations were defined as following: Polymorphonuclear granulocytes: FSC/SSC gating for granulocytes; $Cd14^-$; $CD15^+CD66b^+$; $CD11bhighCD16^+$. Classical monocytes: FSC/SSC gating for monocytes; $CD11b^+$; $CD14^{high}CD16^-$. Intermediate monocytes: FSC/SSC gating for monocytes; $CD11b^+$; $CD14^{high}CD16^+$. Non-classical monocytes: FSC/SSC gating for monocytes; $CD11b^+$; $CD14^-CD16^+$.

### Transcriptomics analysis

To analyze gene expression in peripheral blood mononuclear cells (PBMC), we used 10x single cell RNASeq technology (www.10xgenomics.com/solutions/single-cell/). Isolated leukocytes were analyzed in batch of 6 using the Chromium Next GEM Chip G Single Cell Kit. 15'000 cells are incubated with gel beads (Chromium Next GEM Single Cell 3' GEM, Library & Gel Bead Kit v3.1) on the Chromium Controller & Next GEM Accessory Kit to generate barcoded cDNA in 10'000 cells. The barcoded cDNA was amplified in a and a multiplex code is added for unique identification. Libraries were sequenced using an Illumina HiSeq 2500 platform. To analyze gene expression in PMN we performed bulk sequencing. To this end total RNA was isolated form freshly isolated and lysed PMN and transcribed and used to generated cDNA libraries. Libraries were sequenced using an Illumina HiSeq 2500 platform.

RNA molecules were counted using unique molecular identifiers (UMIs) and normalized to compensate for differences in total transcriptome size between cell types. Sequencing reads were quality trimmed with Cutadapt (v.1.3) and aligned against Homo sapiens.GRCh37.75 genome using TopHat (v.2.0.9. Data normalization and differential expression analysis was performed in R (v.3.1.1), using Bioconductor packages. Dimensionality reduction and data visualization was performed applying methods like the t-distributed Stochastic Network Embedding (t-SNE) based on an appropriate number of principal components.

**Statistical analysis**

Acquired data were analyzed and graphics were generated using Prism Software (GraphPad, La Jolla, CA, USA). Statistical comparisons were performed by T-test assuming non-homogenous variance. Normality distribution of the samples was checked in case of significance and if non-Gaussian a Mann-Whitney replaced the T-test results. Normality distribution of the samples was checked in case of significance and if non-Gaussian a Kruskal-Wallis assay replaced the ANOVA results. Results were considered to be significantly from $p<0.05$. In the figures the various p values thresholds are presented as follow: $\leq 0.05$=*, $\leq 0.01$=**, $\leq 0.001$=***, $\leq 0.0001$=****.

**Example 4**

To select the most relevant biomarkers, applicants used data obtained from healthy donors and patients with primary breast cancer. For each of the protein biomarkers detected by flow cytometry, applicants first obtained a normalized value for each of the subjects. Using these values, applicants tested if the mean value for or each biomarker of the healthy group and for the primary tumor group was the same. To do this, applicants performed a non-parametric bilateral test of mean-comparison (Wilcoxon/Mann-Whitney test. Due to multiple comparisons (one per candidate biomarker), the critical probabilities are corrected according to the Bonferroni method. In order to test the individual discrimination capacity of each biomarker, applicants calculated the performance (false positive rate, false negative rate) of a classification model using only the value of this biomarker as a predictor of the group, at various thresholds. This information is synthesized via the calculation of the Area Under the Curve (AUC) of the receiver operating characteristic (ROC) curve created by plotting the true positive rate (TPR) against the false positive rate (FPR) at various threshold settings of the biomarker value. AUC values are equivalent to Wilcoxon test of ranks. Applicants therefore selected the biomarkers with the lowest critical probability in the Wilcoxon test, and the highest AUC.

Applicants used this biomarker as input variable for a Logistic regression with the two class (healthy, primary tumor) as binary variable to predict. Applicants use group of patients to estimate the parameters of this predictive models.

Table 9

| Biomarker description | Biomarker AUC | P val (Wilcoxon test) | P val_adj (Bonferroni correction | coeff_ estimate |
|---|---|---|---|---|
| Classical Monocytes: Monocyte scatter, CD11b+ CD117+ over Freq of Parent | 0,69 | 5,31E-02 | 8,50E-02 | -551,28 |
| PMN Neutrophils: PMN scatter, CD11b+ CD62L+ Freq of Parent | 0,88 | 1,03E-04 | 6,63E-04 | -56,57 |
| Classical Monocytes: Monocyte scatter, CD11b+ CD202b+ Freq of Parent | 0,85 | 1,66E-04 | 6,63E-04 | -25,45 |
| Intermediate Monocytes: Monocyte scatter CD11b+ CD202b+ Freq of Parent | 0,78 | 4,20E-03 | 8,40E-03 | 7,62 |
| Non-Classical Monocytes, Polarized Monocytes: CD11b+ CD86+ | 0,62 | 2,25E-01 | 2,57E-01 | 9,99 |

(continued)

| Biomarker description | Biomarker AUC | P val (Wilcoxon test) | P val_adj (Bonferroni correction | coeff_ estimate |
|---|---|---|---|---|
| Intermediate Monocytes: Monocyte scatter, CD11b$^+$ CD144$^+$ Freq of Parent | 0,67 | 9,18E-02 | 1,22E-01 | -6,21 |
| Non classical Monocytes: Monocyte scatter CD11b$^+$ CD177$^+$ Freq of Parent | 0,80 | 2,43E-03 | 6,47E-03 | 2,98 |

Table 9 represents for each biomarker (column from left to right); the area under the curve (AUC), the critical probability of a Wilcoxon test (P vs HD) and the estimated value of the corresponding parameter in the logistic model.

Biomarkers consist in the expression of the given CD molecule in the specific cellular subpopulations : Classical, Non classical, Intermediate Monocytes; Neutrophil PMN.

AUC defines the performance of a classification model consisting of this specific biomarker. P val = Critical probability of a test of Wilcoxon (= Mann-Whitney), non-parametric test of comparison of means of two samples. A low value indicates that the value for the biomarker is on average higher in one group than in the other (HD, P).

P val adjust: The same value corrected for multiple tests via the Bonferonni method.

Coeff estimate: Value of the coefficient $\beta$ by which the measured value of the biomarker must be multiplied to have the probability of identifying patients with a primary tumor using the logistic formula.

After estimating the model, applicants applied the model to a set of patients whose condition is known. For each patient, using the values of each biomarker, the model predicts (value between 0 and 1) the risk score that the patient is in the primary tumor group.

$$A = 43.45 - 551.28 \times CD117_{classical} - 56.67 \times CD62L - 25.45 \times CD202b_{classical}$$
$$+ 7.62 \times CD202B_{intermerdiate} + 9.99 \times CD86 + 2.98 \times CD117_{non\ classical}$$
$$- 6.21 \times CD144_{non\ classical}$$

$$P(y_i = 1) = \frac{e^A}{1 - e^A}$$

Knowing the status of each individual (healthy donor vs primary tumor patient), applicants calculated the Area Under the Curve of the ROC curve created by plotting the specificity (1 - FPR) against the sensitivity (TPR) at various threshold settings of the biomarker value, and calculate the AUC for this model. AUC for complete logistic model is 0.97, for a specificity of 0.8 we have a sensitivity of 100%. The resulting plot is shown in Figure 10.

By the way, genetic data namely the expression level of transcriptomic (mRNA) markers of the first panel comprising the combination of SOX4, TNFSF10, CD3G, and NR3C2 are treated in the same way and integrated with FACS data.

## Methods

Calculations, regressions and graphical representation were done using statistical software R-4.1.2 for Windows (32/64 bit) with library glmnet and ROCR.

## References

1. Sotiriou, C., et al., Breast cancer classification and prognosis based on gene expression profiles from a population-based study. Proc Natl Acad Sci U S A, 2003. 100(18): p. 10393-8.

2. Sleeman, J.P., et al., Concepts of metastasis in flux: the stromal progression model. Semin Cancer Biol, 2012. 22(3): p. 174-86.

3. Cardoso, F., et al., 70-Gene Signature as an Aid to Treatment Decisions in Early-Stage Breast Cancer. N Engl J Med, 2016. 375(8): p. 717-29.

4. Lorusso, G. and C. Ruegg, The tumor microenvironment and its contribution to tumor evolution toward metastasis. Histochem Cell Biol, 2008. 130(6): p. 1091-103.

5. Salgado, R., et al., The evaluation of tumor-infiltrating lymphocytes (TILs) in breast cancer: recommendations by an International TILs Working Group 2014. Ann Oncol, 2015. 26(2): p. 259-71.

6. van den Ende, C., et al., Benefits and harms of breast cancer screening with mammography in women aged 40-49 years: A systematic review. Int J Cancer, 2017. 141(7): p. 1295-1306.

7. Alimirzaie, S., M. Bagherzadeh, and M.R. Akbari, Liquid biopsy in breast cancer: A comprehensive review. Clin Genet, 2019. 95(6): p. 643-660.

8. Duffy, M.J., E.W. McDermott, and J. Crown, Blood-based biomarkers in breast cancer: From proteins to circulating tumor cells to circulating tumor DNA. Tumour Biol, 2018. 40(5): p. 1010428318776169.

9. Cattin, S., et al., Bevacizumab specifically decreases elevated levels of circulating KIT+CD11b+ cells and IL-10 in metastatic breast cancer patients. Oncotarget, 2016. 7(10): p. 11137-50.

10. Laurent, J., et al., Proangiogenic factor PIGFprograms CD11b(+) myelomonocytes in breast cancer during differentiation of their hematopoietic progenitors. Cancer research, 2011. 71(11): p. 3781-91.

11. Guex, N., et al., Angiogenic activity of breast cancer patients' monocytes reverted by combined use of systems modeling and experimental approaches. PLoS Comput Biol, 2015. 11(3): p. e1004050.

12. Kuonen, F., et al., Inhibition of the Kit ligand/c-Kit axis attenuates metastasis in a mouse model mimicking local breast cancer relapse after radiotherapy. Clin Cancer Res, 2012. 18(16): p. 4365-74.

**Claims**

1. A detection method for breast cancer in a female subject, comprising:

   (a) measuring in a biologic sample obtained from said female subject the expression level of transcriptomic (mRNA) markers of a first panel comprising the combination of SOX4, TNFSF10, CD3G, and NR3C2 and cell surface biomarkers specific for the immune response elicited by breast cancer at its different stages of a second panel comprising the combination of CD11b, CD62L, CD86, CD117, CD144, CD177, and CD202b;
   (b) calculating a probability score based on the measurement of step (a); and
   (c) ruling out breast cancer for said female subject if the score in step (b) is lower than a pre-determined score; or
   (d) ruling in the likelihood of breast cancer for said female subject if the score in step (b) is higher than a pre-determined score,
   in which the probability score P is calculated by the formula:

$$\mathrm{LOG}\left(\frac{P(y_i = 1)}{1 - P(y_i = 1)}\right) = \beta_0 + \beta_1.x_{1_i} + \cdots + \beta_n.x_{n_i}$$

   where $x_{ni}$, is a measured value for the biomarker n and subject i and $(\beta_0, \beta_1, ..., \beta_n)$ is a vector of coefficients with $\beta_0$ a panel-specific constant, and βn is the corresponding logistic regression coefficient of the biomarker n, and wherein a probability score value $Py_i$ closer to 1.0 represents the likelihood of breast cancer for said female subject and a probability score value $Py_i$ closer to 0.0 represents the likelihood that said female subject has not developed breast cancer.

2. The detection method for breast cancer of claim 1, **characterized in that** the cell surface biomarkers specific for the immune response elicited by breast cancer at its different stages of the second panel further comprises at least one protein biomarkers selected from the group comprising FcεRI, HLA-DR, CD69, CD101, CD163, CD170, and CD274 .

3. The detection method for breast cancer_of any of claims 1-2, **characterized in that** the cell surface biomarkers specific for the immune response elicited by breast cancer at its different stages of the second panel further comprises at least one protein biomarkers selected from the group comprising CD3, CD14, CD16, CD15, CD19, CD20, CD56, and CD66b.

4. The detection method for breast cancer according to any of claims 1 to 3, wherein said transcriptomic (mRNA) markers of said first panel further comprises at least one transcript marker selected from the group comprising FCER1A, GZMH, KLF12, HLA-DOA, CX3CR1, HMGB2, LY9, S1PR1, and KLRB1.

5. The detection method for breast cancer according to any of claims 1 to 4, wherein said transcriptomic (mRNA) markers of said first panel further comprises at least one transcript marker selected from the group comprising TGFBR3, CCL20, CCR3, FN1, ACKR3, IL10, CXCL10, C3, MKI67, HBEGF, C9orf47, CD40, EREG, CXCL9, and SERPINE1.

6. The detection method for breast cancer according to any of claims 1 to 5, wherein said probability score is calculated from a logistic regression prediction model applied to the measurement by an algorithm.

7. Use of a kit in the detection, stratification and/or monitoring of a likelihood of breast cancer in a female subject from a peripheral blood sample, said kit consisting of :

   - probes for measuring the expression level of transcriptomic (mRNA) marker of a first panel of genes consisting in the combination of SOX4, TNFSF10, CD3G, and NR3C2; and
   - probes and/or specific detection reagents for measuring the expression level of cell surface biomarker specific for the immune response elicited by breast cancer at its different stages of a second panel consisting in the combination of CD11b, CD62L, CD86, CD117, CD144, CD177, and CD202b.

8. The use of claim 7, further comprising at least one probe and/or specific detection reagent for measuring one or more cell surface biomarker of the second panel comprising FcεRI, HLA-DR, CD69, CD101, CD163, CD170, and CD274.

9. The use of any of claims 7-8, further comprising at least one probe and/or specific detection reagent for measuring one or more cell surface biomarker of the second panel comprising CD3, CD14, CD16, CD15, CD19, CD20, CD56, and CD66b.

10. The use of any of claims 7-9, further comprising at least one probe for measuring one or more transcriptomic (mRNA) marker of the first panel comprising FCER1A, GZMH, KLF12, HLA- DOA, CX3CR1, HMGB2, LY9, S1PR1, and KLRB1.

11. The use of any of claims 7-10, further comprising at least one probe for measuring one or more transcriptomic (mRNA) marker of the first panel comprising TGFBR3, CCL20, CCR3, FN1, ACKR3, IL10, CXCL10, C3, MKI67, HBEGF, C9orf47, CD40, EREG, CXCL9, and SERPINE1.

12. The detection method for breast cancer according to any of claims 1 to 6, wherein said biologic sample is selected from the group consisting of peripheral blood mononuclear cells, blood cells, whole blood, serum, plasma, circulating tumor cells, lymphatic fluid, bone marrow and cerebrospinal fluid (CSF).

13. The detection method for breast cancer according to any one of claims 1 to 6, wherein said breast cancer is a carcinoma.

14. The detection method for breast cancer according to any one of claims 1 to 6, wherein the likelihood of breast cancer is further determined by the sensitivity, specificity, negative predictive value (NPV) or positive predictive value (PPV) associated with the score.

15. The detection method for breast cancer according to any one of claims 1 to 6, wherein said female subject is at risk of developing primary or recurrent breast cancer.

16. The detection method for breast cancer according to any one of claims 1 to 6, wherein the detection is an early detection or a detection of follow-up breast cancers at any stage of the disease.

17. The use of the kit in the detection of breast cancer of any of claims 7-11, wherein said kit further comprises: primer pairs specific for one or more housekeeping genes selected from the list comprising *TBP, SDHA, ACTBIPO8, HuPO, BA, CYC, GAPDH, PGK, B2M,* GAPDH

**Patentansprüche**

1.  Verfahren zum Nachweis von Brustkrebs bei einer weiblichen Person, umfassend:

    (a) Messen des Expressionsniveaus von Transkriptom-Markern (mRNA) eines ersten Panels, umfassend die Kombination von SOX4, TNFSF10, CD3G und NR3C2, und von Zelloberflächen-Biomarkern, die für die durch Brustkrebs in seinen verschiedenen Stadien ausgelöste Immunantwort spezifisch sind, eines zweiten Panels, umfassend die Kombination von CD11b, CD62L, CD86, CD117, CD144, CD177 und CD202b, in einer biologischen Probe, die von der weiblichen Person erhalten wurde;
    (b) Berechnen eines Wahrscheinlichkeits-Scores basierend auf der Messung von Schritt (a); und
    (c) Ausschließen von Brustkrebs für die weibliche Person, wenn der Score in Schritt (b) niedriger ist als ein vorbestimmter Score; oder
    (d) Bestätigen der Wahrscheinlichkeit von Brustkrebs für die weibliche Person, wenn der Score in Schritt (b) höher ist als ein vorbestimmter Score,
    wobei der Wahrscheinlichkeits-Score P nach der folgenden Formel berechnet wird:

    $$\mathrm{LOG}\left(\frac{P(y_i = 1)}{1 - P(y_i = 1)}\right) = \beta_0 + \beta_1 . x_{1_i} + \cdots + \beta_n . x_{n_i}$$

    wobei $x_{ni}$ ein gemessener Wert für den Biomarker n und das Subjekt i ist und ($\beta_0$, $\beta_1$, ..., $\beta_n$) ein Vektor von Koeffizienten ist, wobei $\beta_0$ eine panelspezifische Konstante ist und $\beta_n$ der entsprechende logistische Regressionskoeffizient des Biomarkers n ist, und wobei ein Wahrscheinlichkeits-Score-Wert $P_{yi}$ näher an 1,0 die Wahrscheinlichkeit von Brustkrebs für die weibliche Person darstellt und ein Wahrscheinlichkeits-Score-Wert $Py_i$ näher an 0,0 die Wahrscheinlichkeit darstellt, dass die weibliche Person keinen Brustkrebs entwickelt hat.

2.  Verfahren zum Nachweis von Brustkrebs nach Anspruch 1, **dadurch gekennzeichnet, dass** die Zelloberflächen-Biomarker, die für die durch Brustkrebs in seinen verschiedenen Stadien ausgelöste Immunantwort spezifisch sind, des zweiten Panels ferner mindestens einen Protein-Biomarker umfassen, ausgewählt aus der Gruppe umfassend FceRI, HLA-DR, CD69, CD101, CD163, CD170 und CD274.

3.  Verfahren zum Nachweis von Brustkrebs nach einem der Ansprüche 1 bis 2, **dadurch gekennzeichnet, dass** die Zelloberflächen-Biomarker, die für die durch Brustkrebs in seinen verschiedenen Stadien ausgelöste Immunantwort spezifisch sind, des zweiten Panels ferner mindestens einen Protein-Biomarker umfassen, ausgewählt aus der Gruppe umfassend CD3, CD14, CD16, CD15, CD19, CD20, CD56 und CD66b.

4.  Verfahren zum Nachweis von Brustkrebs nach einem der Ansprüche 1 bis 3, wobei die Transkriptom-Marker (mRNA) des ersten Panels ferner mindestens einen Transkriptmarker umfassen, ausgewählt aus der Gruppe umfassend FCER1A, GZMH, KLF12, HLA-DOA, CX3CR1, HMGB2, LY9, S1PR1 und KLRB1.

5.  Verfahren zum Nachweis von Brustkrebs nach einem der Ansprüche 1 bis 4, wobei die Transkriptom-Marker (mRNA) des ersten Panels ferner mindestens einen Transkriptmarker umfassen, ausgewählt aus der Gruppe umfassend TGFBR3, CCL20, CCR3, FN1, ACKR3, IL10, CXCL10, C3, MK167, HBEGF, C9orf47, CD40, EREG, CXCL9 und SERPINE1.

6.  Verfahren zum Nachweis von Brustkrebs nach einem der Ansprüche 1 bis 5, wobei der Wahrscheinlichkeits-Score aus einem logistischen Regressionsvorhersagemodell berechnet wird, das auf die Messung durch einen Algorithmus angewendet wird.

7.  Verwendung eines Kits bei der Detektion, Stratifizierung und/oder Überwachung einer Wahrscheinlichkeit von Brustkrebs bei einer weiblichen Person aus einer peripheren Blutprobe, wobei das Kit besteht aus:

    - Sonden zum Messen des Expressionsniveaus von Transkriptom-Markern (mRNA) eines ersten Panels von Genen, bestehend aus der Kombination von SOX4, TNFSF10, CD3G und NR3C2; und

- Sonden und/oder spezifischen Detektionsreagenzien zum Messen des Expressionsniveaus von Zelloberflächen-Biomarkern, die für die durch Brustkrebs in seinen verschiedenen Stadien ausgelöste Immunantwort spezifisch sind, eines zweiten Panels, bestehend aus der Kombination von CD11b, CD62L, CD86, CD117, CD144, CD177 und CD202b.

8. Verwendung nach Anspruch 7, ferner umfassend mindestens eine Sonde und/oder ein spezifisches Detektionsreagenz zum Messen eines oder mehrerer Zelloberflächen-Biomarker des zweiten Panels, umfassend FcεRI, HLA-DR, CD69, CD101, CD163, CD170 und CD274.

9. Verwendung nach einem der Ansprüche 7 bis 8, ferner umfassend mindestens eine Sonde und/oder ein spezifisches Detektionsreagenz zum Messen eines oder mehrerer Zelloberflächen-Biomarker des zweiten Panels, umfassend CD3, CD14, CD16, CD15, CD19, CD20, CD56 und CD66b.

10. Verwendung nach einem der Ansprüche 7 bis 9, ferner umfassend mindestens eine Sonde zum Messen eines oder mehrerer Transkriptom-Marker (mRNA) des ersten Panels, umfassend FCER1A, GZMH, KLF12, HLA-DOA, CX3CR1, HMGB2, LY9, S1PR1 und KLRB1.

11. Verwendung nach einem der Ansprüche 7 bis 10, ferner umfassend mindestens eine Sonde zum Messen eines oder mehrerer Transkriptom-Marker (mRNA) des ersten Panels, umfassend TGFBR3, CCL20, CCR3, FN1, ACKR3, IL10, CXCL10, C3, MK167, HBEGF, C9orf47, CD40, EREG, CXCL9 und SERPINE1.

12. Verfahren zum Nachweis von Brustkrebs nach einem der Ansprüche 1 bis 6, wobei die biologische Probe ausgewählt ist aus der Gruppe bestehend aus peripheren mononukleären Blutzellen, Blutzellen, Vollblut, Serum, Plasma, zirkulierenden Tumorzellen, Lymphe, Knochenmark und Liquor cerebrospinalis (LCS).

13. Verfahren zum Nachweis von Brustkrebs nach einem der Ansprüche 1 bis 6, wobei der Brustkrebs ein Karzinom ist.

14. Verfahren zum Nachweis von Brustkrebs nach einem der Ansprüche 1 bis 6, wobei die Wahrscheinlichkeit von Brustkrebs ferner durch die Sensitivität, Spezifität, den negativen Vorhersagewert (NPV) oder den positiven Vorhersagewert (PPV) bestimmt wird, die mit dem Score verbunden sind.

15. Verfahren zum Nachweis von Brustkrebs nach einem der Ansprüche 1 bis 6, wobei die weibliche Person gefährdet ist, primären oder wiederkehrenden Brustkrebs zu entwickeln.

16. Verfahren zum Nachweis von Brustkrebs nach einem der Ansprüche 1 bis 6, wobei die Detektion eine Früherkennung oder eine Erkennung von Folge-Brustkrebs in jedem Stadium der Erkrankung ist.

17. Verwendung des Kits bei der Detektion von Brustkrebs nach einem der Ansprüche 7 bis 11, wobei das Kit ferner umfasst: Primerpaare, die spezifisch für ein oder mehrere Housekeeping-Gene sind, ausgewählt aus der Liste umfassend TBP, SDHA, ACTBIP08, HuPO, BA, CYC, GAPDH, PGK, B2M, GAPDH.

**Revendications**

1. Procédé de détection du cancer du sein chez une femme, comprenant :

   (a) la mesure dans un échantillon biologique obtenu de ladite femme du niveau d'expression de marqueurs transcriptomiques (ARNm) d'un premier panel comprenant la combinaison de SOX4, TNFSF10, CD3G et NR3C2, et de biomarqueurs de surface cellulaire spécifiques de la réponse immunitaire induite par le cancer du sein à ses différents stades d'un second panel comprenant la combinaison de CD11b, CD62L, CD86, CD117, CD144, CD177 et CD202b ;
   (b) le calcul d'un score de probabilité sur la base de la mesure de l'étape (a) ; et
   (c) l'exclusion du cancer du sein pour ladite femme si le score de l'étape (b) est inférieur à un score prédéterminé ; ou
   (d) conclure à la probabilité d'un cancer du sein pour ladite femme si le score de l'étape (b) est supérieur à un score prédéterminé,
   dans lequel le score de probabilité P est calculé par la formule :

$$\text{LOG}\left(\frac{P(y_i = 1)}{1 - P(y_i = 1)}\right) = \beta_0 + \beta_1.x_{1_i} + \cdots + \beta_n.x_{n_i}$$

où $x_{ni}$ est une valeur mesurée pour le biomarqueur n et le sujet i, et $(\beta_0, \beta_1, ..., \beta_n)$ est un vecteur de coefficients, $\beta_0$ étant une constante propre au panel, et $\beta n$ étant le coefficient de régression logistique correspondant du biomarqueur n, et dans lequel une valeur du score de probabilité $Py_i$ plus proche de 1,0 représente la probabilité d'un cancer du sein pour ladite femme et une valeur du score de probabilité $Py_i$ plus proche de 0,0 représente la probabilité que ladite femme n'ait pas développé de cancer du sein.

2. Procédé de détection du cancer du sein selon la revendication 1, **caractérisé en ce que** les biomarqueurs de surface cellulaire spécifiques de la réponse immunitaire induite par le cancer du sein à ses différents stades du second panel comprennent en outre au moins un biomarqueur protéique choisi dans le groupe comprenant FcεRI, HLA-DR, CD69, CD101, CD163, CD170 et CD274.

3. Procédé de détection du cancer du sein selon l'une quelconque des revendications 1 à 2, **caractérisé en ce que** les biomarqueurs de surface cellulaire spécifiques de la réponse immunitaire induite par le cancer du sein à ses différents stades du second panel comprennent en outre au moins un biomarqueur protéique choisi dans le groupe comprenant CD3, CD14, CD16, CD15, CD19, CD20, CD56 et CD66b.

4. Procédé de détection du cancer du sein selon l'une quelconque des revendications 1 à 3, dans lequel lesdits marqueurs transcriptomiques (ARNm) dudit premier panel comprennent en outre au moins un marqueur transcriptionnel choisi dans le groupe comprenant FCER1A, GZMH, KLF12, HLA-DOA, CX3CR1, HMGB2, LY9, S1PR1 et KLRB1.

5. Procédé de détection du cancer du sein selon l'une quelconque des revendications 1 à 4, dans lequel lesdits marqueurs transcriptomiques (ARNm) dudit premier panel comprennent en outre au moins un marqueur transcriptionnel choisi dans le groupe comprenant TGFBR3, CCL20, CCR3, FN1, ACKR3, IL10, CXCL10, C3, MKI67, HBEGF, C9orf47, CD40, EREG, CXCL9 et SERPINE1.

6. Procédé de détection du cancer du sein selon l'une quelconque des revendications 1 à 5, dans lequel ledit score de probabilité est calculé à partir d'un modèle de prédiction par régression logistique appliqué à la mesure par un algorithme.

7. Utilisation d'un kit dans la détection, la stratification et/ou la surveillance d'une probabilité de cancer du sein chez une femme à partir d'un échantillon de sang périphérique, ledit kit étant constitué de :

   - sondes pour mesurer le niveau d'expression de marqueurs transcriptomiques (ARNm) d'un premier panel de gènes constitué de la combinaison de SOX4, TNFSF10, CD3G et NR3C2 ; et de
   - sondes et/ou réactifs de détection spécifiques pour mesurer le niveau d'expression de biomarqueurs de surface cellulaire spécifiques de la réponse immunitaire induite par le cancer du sein à ses différents stades d'un second panel constitué de la combinaison de CD11b, CD62L, CD86, CD117, CD144, CD177 et CD202b.

8. Utilisation selon la revendication 7, comprenant en outre au moins une sonde et/ou un réactif de détection spécifique pour mesurer un ou plusieurs biomarqueurs de surface cellulaire du second panel comprenant FcεRI, HLA-DR, CD69, CD101, CD163, CD170et CD274.

9. Utilisation selon l'une quelconque des revendications 7 à 8, comprenant en outre au moins une sonde et/ou un réactif de détection spécifique pour mesurer un ou plusieurs biomarqueurs de surface cellulaire du second panel comprenant CD3, CD14, CD16, CD15, CD19, CD20, CD56 et CD66b.

10. Utilisation selon l'une quelconque des revendications 7 à 9, comprenant en outre au moins une sonde pour mesurer un ou plusieurs marqueurs transcriptomiques (ARNm) du premier panel comprenant FCER1A, GZMH, KLF12, HLA-DOA, CX3CR1, HMGB2, LY9, S1PR1 et KLRB1.

11. Utilisation selon l'une quelconque des revendications 7 à 10, comprenant en outre au moins une sonde pour mesurer un ou plusieurs marqueurs transcriptomiques (ARNm) du premier panel comprenant TGFBR3, CCL20, CCR3, FN1, ACKR3, IL10, CXCL10, C3, MK167, HBEGF, C9orf47, CD40, EREG, CXCL9 et SERPINE1.

**12.** Procédé de détection du cancer du sein selon l'une quelconque des revendications 1 à 6, dans lequel ledit échantillon biologique est choisi dans le groupe constitué de cellules mononucléées du sang périphérique, de cellules sanguines, de sang total, de sérum, de plasma, de cellules tumorales circulantes, de liquide lymphatique, de moelle osseuse et de liquide céphalo-rachidien (LCR).

**13.** Procédé de détection du cancer du sein selon l'une quelconque des revendications 1 à 6, dans lequel ledit cancer du sein est un carcinome.

**14.** Procédé de détection du cancer du sein selon l'une quelconque des revendications 1 à 6, dans lequel la probabilité de cancer du sein est en outre déterminée par la sensibilité, la spécificité, la valeur prédictive négative (VPN) ou la valeur prédictive positive (VPP) associée au score.

**15.** Procédé de détection du cancer du sein selon l'une quelconque des revendications 1 à 6, dans lequel ladite femme est susceptible de développer un cancer du sein primaire ou récurrent.

**16.** Procédé de détection du cancer du sein selon l'une quelconque des revendications 1 à 6, dans lequel la détection est une détection précoce ou une détection de suivi des cancers du sein à tout stade de la maladie.

**17.** Utilisation du kit dans la détection du cancer du sein selon l'une quelconque des revendications 7 à 11, ledit kit comprenant en outre : des paires d'amorces spécifiques d'un ou plusieurs gènes de ménage choisis dans la liste comprenant TBP, SDHA, ACTBIP08, HuPO, BA, CYC, GAPDH, PGK, B2M, GAPDH.

FIG: 1

FIG: 2

**FIG: 3**

1. Data acquisition

1. Compensation
2. Debris and dead cell removal
3. Doublets removal
4. FlowCLEAN

1. Downsampling
2. Concatenate
3. FlowSOM

1. Group separation
2. Cluster definition
3. Results

**FIG: 4**

**FIG: 5**

**FIG: 6**

**FIG: 7**

**FIG: 8**

**FIG: 9**

**FIG: 10**

AUC: 0.79

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014068124 A1
- WO 2017011559 A1
- WO 2008123867 A1, WASSMANN KARL

### Non-patent literature cited in the description

- **NAGARAJAN DIVYA et al.** Immune Landscape of Breast Cancers. *Biomedicines*, 11 February 2018, vol. 6 (1), 20
- The tumor microenvironment and its contribution to tumor evolution toward metastasis. **GIRIECA LORUSSO et al.** histochemistry and cell biology. Springer, 06 November 2008, vol. 130, 1091-1103
- **B. RUFFELL et al.** Leukocyte composition of human breast cancer. *Proceedings of the National Academy of Sciences*, 08 August 2011, vol. 109 (8), ISSN 0027-8424, 2796-2801
- SOX4 overexpression is a novel biomarker of malignant status and poor prognosis in breast cancer patients. **SONG GUO-DONG et al.** TUMOR BIOLOGY. KARGER, 16 January 2015, vol. 36, 4167-4173
- **PARK M. Y** ; **HASTIE T**. An L1 Regularization-path Algorithm for Generalized Linear Models. *A generalization of the LARS algorithm for GLMs and the Cox proportional hazard model*, 2006
- **GOEMAN, J.** L1 (lasso) and L2 (ridge) penalized estimation. *GLMs and in the Cox model*, 2010
- **HASTI, T.** ; **TIBSHIRANI AND R.** ; **FRIEDMAN, J.** *Lasso and elastic-net regularized generalized linear models*, 2010
- Clinical Interpretation Of Laboratory Procedures. **SHULTZ**. Fundamentals of Clinical Chemistry. W.B. Saunders Company, 1996, 192-199
- **ZWEIG et al.** *ROC Curve Analysis: An ROC curve is an x-y plot of sensitivity on the y-axis*
- *Am. J. Public Health*, vol. 86 (5), 628-629
- **SOTIRIOU, C. et al.** Breast cancer classification and prognosis based on gene expression profiles from a population-based study. *Proc Natl Acad Sci U S A*, 2003, vol. 100 (18), 10393-8
- **SLEEMAN, J.P et al.** Concepts of metastasis in flux: the stromal progression model. *Semin Cancer Biol*, 2012, vol. 22 (3), 174-86
- **CARDOSO, F. et al.** 70-Gene Signature as an Aid to Treatment Decisions in Early-Stage Breast Cancer. *N Engl J Med*, 2016, vol. 375 (8), 717-29
- **LORUSSO, G.** ; **C. RUEGG**. The tumor microenvironment and its contribution to tumor evolution toward metastasis. *Histochem Cell Biol*, 2008, vol. 130 (6), 1091-103
- **SALGADO, R. et al.** The evaluation of tumor-infiltrating lymphocytes (TILs) in breast cancer: recommendations by an International TILs Working Group 2014. *Ann Oncol*, 2015, vol. 26 (2), 259-71
- **VAN DEN ENDE, C. et al.** Benefits and harms of breast cancer screening with mammography in women aged 40-49 years: A systematic review. *Int J Cancer*, 2017, vol. 141 (7), 1295-1306
- **ALIMIRZAIE, S.** ; **M. BAGHERZADEH** ; **M.R. AKBARI**. Liquid biopsy in breast cancer: A comprehensive review. *Clin Genet*, 2019, vol. 95 (6), 643-660
- **DUFFY, M.J.** ; **E.W. MCDERMOTT** ; **J. CROWN**. Blood-based biomarkers in breast cancer: From proteins to circulating tumor cells to circulating tumor DNA. *Tumour Biol*, 2018, vol. 40 (5), 1010428318776169
- **CATTIN, S. et al.** Bevacizumab specifically decreases elevated levels of circulating KIT+CD11b+ cells and IL-10 in metastatic breast cancer patients. *Oncotarget*, 2016, vol. 7 (10), 11137-50
- **LAURENT, J. et al.** Proangiogenic factor PIGFprograms CD11b(+) myelomonocytes in breast cancer during differentiation of their hematopoietic progenitors. *Cancer research*, 2011, vol. 71 (11), 3781-91
- **GUEX, N. et al.** Angiogenic activity of breast cancer patients' monocytes reverted by combined use of systems modeling and experimental approaches. *PLoS Comput Biol*, 2015, vol. 11 (3), e1004050
- **KUONEN, F. et al.** Inhibition of the Kit ligand/c-Kit axis attenuates metastasis in a mouse model mimicking local breast cancer relapse after radiotherapy. *Clin Cancer Res*, 2012, vol. 18 (16), 4365-74